# DEMANDE DE BREVET EUROPEEN

(11) **EP 2 039 346 A1**
(43) Date de publication de la demande: **25.03.2009**
(21) Numéro de dépôt: 08164236.5
(22) Date de dépôt: 12.09.2008
(51) Int. Cl.: A61K 8/81, A61K 8/73, A61Q 5/02, A61Q 5/12

(54) **Composition cosmétique comprenant un copolymère cationique et un amidon et procédé de traitement cosmétique**

(30) Priorité: 14.09.2007 FR 0757584
(71) Demandeur: L'Oréal, 75008 Paris (FR)
(72) Inventeur: Biganska, Olga, 92600 Asnières sur Seine (FR); Chesneau, Laurent, 92300 LEVALLOIS PERRET (FR)
(74) Mandataire: Le Blainvaux Bellegarde, Françoise

(57) **Abrégé**

L'invention concerne de nouvelles compositions cosmétiques comprenant dans un milieu cosmétiquement acceptable :
(i)-un ou plusieurs polymères cationiques qui sont obtenus par polymérisation d'un mélange de monomères comprenant :
a) un ou plusieurs monomères vinyliques substitués par un ou plusieurs groupes amino,
b) un ou plusieurs monomères vinyliques non ioniques hydrophobes,
choisis parmi ceux de formules (I) et (II) :

(I) CH₂=C(X)Z,

(II) CH₂=CH-OC(O)R;

dans lesquelles formules (I) et (II) :
X représente H ou un groupe méthyle ;
Z est choisi parmi les groupes -C(O)OR¹, -C(O)NH₂, -C(O)NHR¹, - C(O)N(R¹)₂, -C₆H₅, -C₆H₄R¹, -C₆H₄OR¹, -C₆H₄Cl, -CN, -NHC(O)CH₃, -NHC(O)H, N-(2-pyrrolidonyle), N-caprolactamyle, -C(O)NHC(CH₃)₃, -C(O)NHCH₂CH₂-NH-CH₂CH₂-urée, -SiR₃, -C(O)O(CH₂)ₓSiR₃, -C(O)NH(CH₂)ₓSiR₃ et -(CH₂)ₓSiR₃ ;
x représente un nombre entier allant de 1 à 6 ;
chaque R représente indépendamment un groupe alkyle en C₁-C₃₀ ; chaque R1 représente indépendamment un groupe alkyle en C1-C30, un groupe alkyle en C2-C30 hydroxylé, ou un groupe alkyle en C1-C30 halogéné et
c) un ou plusieurs monomères vinyliques associatifs,
e) un ou plusieurs monomères vinyliques non ioniques hydroxylés, (ii) un ou plusieurs amidons.

Ces compositions ont une texture qui n'évolue pas au cours du temps et qui permettent une meilleure répartition, de meilleures propriétés moussantes ainsi que de meilleures propriétés cosmétiques.

## Description

La présente invention est relative à une composition cosmétique notamment de conditionnement des cheveux comprenant au moins un amidon modifié ou non modifié, au moins un copolymère cationique et à un procédé de traitement cosmétique des matières kératiniques en particulier les cheveux.

Le plus souvent, un shampoing ou un après-shampooing se présente sous la forme d'un liquide plus ou moins visqueux. On recherche des produits dont la texture soit suffisamment épaisse, pour rester sur les cheveux, sans s'écouler, pendant un certain temps. Cette texture plus épaisse, voire même gélifiée, ne doit pas altérer ses qualités d'usage.

L'épaississement et/ou la gélification des milieux aqueux par des polymères est depuis longtemps un important de sujet de recherche cosmétique. L'obtention d'un effet d'épaississement intéressant par un polymère hydrosoluble suppose généralement une masse molaire élevée et un volume hydrodynamique important. La gélification d'un milieu aqueux est alors considérée comme le résultat d'un réseau polymère tridimensionnel obtenu par réticulation de polymères linéaires ou par copolymérisation de monomères bifonctionnels et polyfonctionnels. L'utilisation de tels polymères de masse molaire très élevée pose cependant un certain nombre de problèmes tels que la texture peu agréable et la difficulté d'étalement des gels obtenus.

On a déjà proposé pour le traitement des matières kératiniques et en particulier des cheveux, des compositions cosmétiques épaissies par des polysaccharides épaississants tels que notamment l'amidon ou les celluloses.

De telles compositions présentent cependant des inconvénients tels que des problèmes de rinçabilité, des problèmes de stabilité, en particulier de synérèse, des difficultés de répartition sur les matières kératiniques ainsi que des propriétés cosmétiques insuffisantes.

En résumé, il s'avère que les compositions cosmétiques actuelles de conditionnement comprenant de l'amidon, ne donnent pas complètement satisfaction. Ainsi, on cherche à obtenir des compositions cosmétiques ayant de très bonnes propriétés cosmétiques en particulier sur des cheveux très sensibilisés, de bonnes propriétés d'usage (bonne répartition, mousse, bon rinçage) ainsi qu'une bonne stabilité.

La demanderesse a maintenant découvert que l'association d'un amidon et d'un copolymère cationique particulier permet de remédier à ces inconvénients.

Les compositions selon l'invention apportent plus de masse, plus de corps et plus brillance aux cheveux par rapport à des compositions comprenant un amidon.

Ces compositions ont une texture qui n'évolue pas ou peu au cours du temps à température ambiante (environ 25°C) et à 45°C. La stabilité des compositions est donc satisfaisante à température ambiante et 45°C, en particulier les compositions selon l'invention ne présentent plus l'effet de synérèse. Ces compositions permettent une meilleure répartition du produit lors de l'application. Lorsque les compositions comprennent des tensioactifs moussants leurs propriétés moussantes, en particulier le démarrage de mousse, sont améliorées.

Ainsi, selon la présente invention, il est maintenant proposé de nouvelles compositions cosmétiques, comprenant dans un milieu aqueux cosmétiquement acceptable :
(i) un ou plusieurs polymères cationiques qui sont produits par polymérisation d'un mélange de monomères comprenant :
   a) un ou plusieurs monomères vinyliques substitués par un ou plusieurs groupes amino,
   b) un ou plusieurs monomères vinyliques non ioniques hydrophobes,
      choisis parmi ceux de formules (I) et (II) :

      (I) CH₂=C(X)Z,

      (II) CH₂=CH-OC(O)R;

      dans lesquelles formules (I) et (II):
      X représente H ou un groupe méthyle;
      Z est choisi parmi les groupes -C(O)OR¹, -C(O)NH₂, -C(O)NHR¹, - C(O)N(R¹)₂, -C₆H₅, -C₆H₄R₁, -C₆H₄OR¹, -C₆H₄Cl, -CN, -NHC(O)CH₃, -NHC(O)H, N-(2-pyrrolidonyle), N-caprolactamyle, -C(O)NHC(CH₃)₃, -C(O)NHCH₂CH₂-NH-CH₂CH₂-urée, -SiR₃, -C(O)O(CH₂)ₓSiR₃, -C(O)NH(CH₂)ₓSiR₃ et -(CH₂)ₓSiR₃;
      x représente un nombre entier allant de 1 à 6 ;
      chaque R représente indépendamment un groupe alkyle en C₁-C₃₀ ;
      chaque R¹ représente indépendamment un groupe alkyle en C₁-C₃₀ , un groupe alkyle en C₂-C₃₀ hydroxylé, ou un groupe alkyle en C₁-C₃₀ halogéné et
   c) un ou plusieurs monomères vinyliques associatifs,
   e) un ou plusieurs monomères vinyliques non ioniques hydroxylés,
      et
(ii) un ou plusieurs amidons

Un autre objet de l'invention consiste en un procédé de traitement cosmétique des matières kératiniques, en particulier les cheveux mettant en oeuvre la composition susvisée.

L'invention a encore pour objet l'utilisation de ladite composition comme shampooing et de préférence comme après-shampoing.

D'autres objets, caractéristiques, aspects et avantages de l'invention apparaîtront encore plus clairement à la lecture de la description et des divers exemples qui suivent.

Par cheveux sensibilisés, on comprend selon la présente invention, des cheveux ayant subi des agressions extérieures physiques (lumière, chaleur, ondes...), des agressions mécaniques (brushing, peignages ou brossages répétés...) ou des agressions chimiques (coloration d'oxydation, décoloration, permanente, défrisage...). Parmi ces agressions, on notera le caractère particulièrement délétère des agressions chimiques. Les compositions selon l'invention sont particulièrement efficaces sur les cheveux sensibilisés par des agressions chimiques.

Par "milieu cosmétiquement acceptable", on entend un milieu compatible avec toutes les matières kératiniques telles que la peau, les cheveux, les ongles, les cils, les sourcils, les lèvres et toute autre zone du corps et du visage.

Par monomère hydrophobe, on entend au sens de la présente invention, un monomère qui présente une solubilité dans l'eau inférieure à 10g pour 100 mL d'eau à une température de 20°C.

L'une des caractéristiques essentielles de l'invention est la présence d'un polymère cationique qui est obtenu par polymérisation d'un mélange de monomères comprenant a) un ou plusieurs monomères vinyliques substitués par un ou plusieurs groupes amines, b) un ou plusieurs monomères vinyliques non ioniques hydrophobes, c) un ou plusieurs monomères vinyliques associatifs et e) un ou plusieurs monomères vinyliques non ioniques hydroxylésmonomères vinyliques non ioniques hydroxylésmonomères vinyliques non ioniques hydroxylésmonomères vinyliques non ioniques hydroxylés . De préférence, les monomères constituant le copolymère cationique comprennent en outre un ou plusieurs monomères tensioactifs vinyliques semi-hydrophobes d). Les monomères a) à e) sont différents les uns des autres.
les monomères a), b), c) et d) étant différents les uns des autres
De préférence, le polymère cationique (i) est un polymère épaississant.

Au sens de la présente invention, on entend par polymère épaississant, un polymère qui, introduit à 1 % en poids dans une solution aqueuse ou hydroalcoolique à 30 % en poids d'éthanol, et à pH 7, permet d'atteindre une viscosité d'au moins 100 cps à 25°C, et à un taux de cisaillement de 1 s⁻¹. Cette viscosité peut être mesurée à l'aide d'un viscosimètre à géométrie cône-plan, par exemple, un rhéomètre Haake RS 600. De préférence, ces polymères permettent d'augmenter la viscosité des compositions dans lesquelles ils se trouvent, d'au moins 50 cps à 25°C et à 1 s⁻¹.

Le ou les polymères cationiques (i) utilisés dans la composition selon l'invention, et leur procédé de fabrication, sont notamment décrits dans la demande internationale WO 2004/024779.

Par monomère vinylique, on entend au sens de la présente invention un monomère comprenant un ou plusieurs groupes R₀CH=C(R₀)- , ou chaque R₀ est indépendamment H, un alkyle en C₁-C₃₀, -C(O)OH ou C(O)OR₀' , -O-C(O)OR₀' , - C(O)NHR₀', C(O)NRo' R₀" ,R₀' et R₀", identiques ou différents, étant un groupe alkyle en C₁-C₃₀.

Ainsi, par exemple, au sens de la présente invention, les (méth)acrylates et les (méth)acrylamides sont des monomères vinyliques.

Comme expliqué précédemment, le mélange de monomères permettant la préparation du polymère cationique (i) utilisé dans la composition selon l'invention comprend un ou plusieurs monomères vinyliques substitués par un ou plusieurs groupes amino.

Les monomères vinyliques substitués par un ou plusieurs groupes amino utilisables pour la préparation du polymère cationique utilisé selon l'invention sont des monomères à insaturation éthylénique, basiques et polymérisables. Les groupes amines peuvent dériver de groupes alkyles mono, di- ou polyaminés, ou bien de groupes hétéroaromatiques comprenant un atome d'azote. Les groupes amines peuvent être des amines primaires, secondaires ou tertiaires. Ces monomères peuvent être utilisés sous la forme d'amine ou sous la forme de sel.

De préférence, le ou les monomères vinyliques substitués par un ou plusieurs groupes amines sont choisis parmi :
- les mono-(C₁-C₄)alkylamino(C₁-C₈)alkyl(méth)acrylates,
- les di-(C₁-C₄)alkylamino(C₁-C₈)alkyl(méth)acrylates, de préférence les di-(C₁-C₄)alkylamino(C₁-C₆)alkyl(méth)acrylates,
- les mono-(C₁-C₄)alkylamino(C₁-C₈)alkyl(méth)acrylamides,
- les di-(C₁-C₄)alkylamino(C₁-C₈)alkyl(méth)acrylamides,
- les (méth)acrylamides à groupement(s) hétérocyclique(s) contenant un atome d'azote,
- les (méth)acrylates à groupement(s) hétérocyclique(s) contenant un atome d'azote,
- les hétérocycles azotés à groupement(s) vinyle(s),
et leurs mélanges.

A titre de monomères vinyliques substitués par un ou plusieurs groupes amino préférés, on peut citer :
- les (méth)acrylates de mono- ou di-(alkyl en C₁-C₄)amino(alkyle en C₁-C₄), tels que le (méth)acrylate de 2-(N,N-diméthylamino)éthyle, le (méth)acrylate de 3-(N,N-diméthylamino)propyle, le (méth)acrylate de 4-(N,N-diméthylamino)butyle, le (méth)acrylate de (N,N-diméthylamino)-t-butyle, le (méth)acrylate de 2-(N,N-diéthylamino)éthyle, le (méth)acrylate de 3-(N,N-diéthylamino)propyle, le (méth)acrylate de 4-(N,N-diéthylamino)butyle, le (méth)acrylate de 2-(N,N-dipropylamino)éthyle, le (méth)acrylate de 3-(N,N-dipropylamino)propyle et le (méth)acrylate de 4-(N,N-dipropylamino)butyle;
- les mono- ou di-(alkyl en C₁-C₄)amino(alkyl en C₁-C₄)-(méth)acrylamides tels que le N'-(2-N,N-diméthylamino)éthyl (méth)acrylamide et le N'-(3-N,N-diméthylamino)propyl acrylamide ;
- les (méth)acrylamides ou (méth)acrylates à groupement hétérocyclique comprenant un atome d'azote, tels que le N-(2-pyridyl)acrylamide, le N-(2-imidazolyl)méthacrylamide, le méthacrylate de 2-(4-morpholinyl)éthyle, le acrylate de 2-(4-morpholinyl)éthyle, le N-(4-morpholinyl)méthacrylamide et le N-(4-morpholinyl)acrylamide; et
- les hétérocycles azotés à groupement(s) vinyle(s) tels que la 2-vinylpyridine et la 4-vinylpyridine.

Lorsque les monomères sont sous forme de sels, il peut s'agir de sels minéraux, tels que les sels hydrochlorure, sulfate et phosphate ; ou bien de sels d'acides organiques, tels que les sels acétate, maléate et fumarate.

Des monomères vinyliques substitués par un ou plusieurs groupe(s) amino particulièrement préférés sont :
- le (méth)acrylate de 3-(N,N-diméthylamino)propyle,
- le N'-(3-N,N-diméthylamino)propyl(méth)acrylamide,
- le (méth)acrylate de 2-(N,N-diméthylamino)éthyle,
- le (méth)acrylate de 2-(N,N-diéthylamino)éthyle,
- le (méth)acrylate de 2-(tert-butylamino)éthyle,
- le 2-(N,N-diméthylamino)propyl(méth)acrylamide, et
- l'acrylate de 2-(N,N-diméthylamino)néopentyle.

Le ou les monomères vinyliques substitués par un ou plusieurs groupes amino représentent généralement de 10 à 70 % en poids, de préférence de 20 à 60 % en poids, mieux de 30 à 40 % en poids, par rapport au poids total du mélange de monomères.

Comme expliqué précédemment, le mélange de monomères permettant la préparation du polymère cationique utilisé (i) selon l'invention comprend également un ou plusieurs monomères vinyliques non ioniques hydrophobes b).

Le ou les monomères vinyliques non ioniques hydrophobes utilisables pour la préparation du polymère cationique utilisé selon l'invention sont généralement choisis parmi les composés de formules (I) et (II):

(I) CH₂=C(X)Z,

(II) CH₂=CH-OC(O)R;

où, dans chacune des formule (I) et (II) ;
X représente H ou un groupe méthyle ;
Z est choisi parmi les groupes -C(O)OR¹, -C(O)NH₂, -C(O)NHR¹, - C(O)N(R¹)₂, -C₆H₅, -C₆H₄R¹, -C₆H₄OR¹, -C₆H₄Cl, -CN, -NHC(O)CH₃, -NHC(O)H, N-(2-pyrrolidonyle), N-caprolactamyle, -C(O)NHC(CH₃)₃, -C(O)NHCH₂CH₂-NH-CH₂CH₂-urée, -SiR₃, -C(O)O(CH₂)ₓSiR₃, -C(O)NH(CH₂)ₓSiR₃ et -(CH₂)ₓSiR₃ ;
x représente un nombre entier allant de 1 à 6 ;
chaque R représente indépendamment un groupe alkyle en C₁-C₃₀ ;
chaque R¹ représente indépendamment un groupe alkyle en C₁-C₃₀ , un groupe alkyle en C₂-C₃₀ hydroxylé, ou un groupe alkyle en C₁-C₃₀ halogéné.

On peut citer en particulier les (méth)acrylates d'alkyle en C₁-C₃₀ ; les (alkyl en C₁-C₃₀)(méth)acrylamides ; le styrène, les styrènes substitués et en particulier le vinyltoluène (ou 2-méthylstyrène), le butylstyrène, l'isopropylstyrène, le para-chlorostyrène ; les esters de vinyle et en particulier l'acétate de vinyle, le butyrate de vinyle, le caprolate de vinyle, le pivalate de vinyle et le néodécanoate de vinyle ; les nitriles insaturés et en particulier le (méth)acrylonitrile et l'acrylonitrile ; et les silanes insaturés et en particulier le triméthylvinylsilane, le diméthyléthylvinylsilane, l'allyldiméthylphénylsilane, l'allyltriméthylsilane, le 3-acrylamidopropyltriméthylsilane, le méthacrylate de 3-triméthylsilylpropyle.

De préférence, le ou les monomères vinyliques non ioniques hydrophobes sont choisis parmi les acrylates d'alkyle en C₁-C₃₀, les méthacrylates d'alkyle en C₁-C₃₀, et leurs mélanges, tels que l'acrylate d'éthyle, le méthacrylate de méthyle, le méthacrylate de 3,3,5-triméthylcyclohexyle.

Le ou les monomères vinyliques non ioniques hydrophobes représentent généralement de 20 à 80% en poids, de préférence de 20 à 70% en poids, mieux de 50 à 65 % en poids, par rapport au poids total du mélange de monomères.

Le ou les monomères vinyliques associatifs utilisables pour la préparation du polymère cationique (i) utilisé selon l'invention sont généralement choisis parmi des composés ayant une extrémité (i)' à insaturation(s) éthylénique(s) pour la polymérisation par addition avec d'autres monomères du système ; une portion centrale (ii)' polyoxyalkylène pour conférer des propriétés hydrophiles sélectives aux polymères, et une extrémité (iii)' hydrophobe pour conférer des propriétés hydrophobes sélectives aux polymères.

L'extrémité (i)' à insaturation(s) éthylénique(s) du ou des monomères vinyliques associatifs est de préférence dérivée d'un acide ou d'un anhydride mono ou di-carboxylique à insaturation(s) α, β-éthylénique(s), de préférence un acide ou un anhydride mono ou di-carboxylique en C₃ ou C₄. De façon alternative, l'extrémité (i)' du monomère associatif peut être dérivée d'un allyl éther ou d'un vinyl éther ; d'un monomère non ionique uréthane substitué par un groupe vinyle, tel que divulgué dans le brevet US redélivré n°33,156 ou dans le brevet US 5,294,692 ; ou d'un produit de réaction urée substituée par un groupe vinyle, tel que divulgué dans le brevet US 5,011,978.

La portion centrale (ii)' du ou des monomères vinyliques associatifs est de préférence un segment polyoxyalkylène comprenant 5 à 250, de préférence encore 10 à 120, et mieux 15 à 60 unités oxydes d'alkylène en C₂-C₇. Des portions centrales (ii)' préférées sont les segments polyoxyéthylène, polyoxypropylène, et polyoxybutylène comprenant 5 à 150, de préférence 10 à 100, et mieux 15 à 60 unités oxydes d'éthylène, de propylène ou de butylène, et des séquences aléatoires ou non aléatoires d'unités oxydes d'éthylène, oxydes de propylène ou oxydes de butylène. De préférence, les portions centrales sont des segments polyoxyéthylène.

L'extrémité (iii)' hydrophobe du ou des monomères associatifs est de préférence un fragment hydrocarboné appartenant à l'une des classes hydrocarbonées suivantes : un alkyle linéaire en C₈-C₄₀, un alkyle en C₂-C₄₀ substitué par un groupe aryle, un phényle substitué par un groupe alkyle en C₂-C₄₀, un alkyle ramifié en C₈-C₄₀, un groupe alicyclique en C₈-C₄₀, et un ester complexe en C₈-C₈₀.

Par ester complexe, on entend au sens de la présente invention tout ester différent d'un ester simple.

Par ester simple, on entend au sens de la présente invention tout ester d'alcool aliphatique saturé en C₁-C₃₀, linéaire ou ramifié, et non substitué.

Des exemples d'extrémités (iii)' hydrophobes du ou des monomères associatifs sont des groupes alkyles linéaires ou ramifiés ayant de 8 à 40 atomes de carbone, tels que les groupes capryle (C₈), isooctyle (C₈ ramifié), décyle (C₁₀), lauryle (C₁₂), myristyle (C₁₄), cétyle (C₁₆), cétéaryle (C₁₆-C₁₈), stéaryle (C₁₈), isostéaryle (C₁₈ ramifié), arachidyle (C₂₀), béhényle (C₂₂), lignocéryle (C₂₄), cérotyle (C₂₆), montanyle (C₂₈), mélyssile (C₃₀) et laccéryle (C₃₂).

Des exemples de groupes alkyles linéaires ou ramifiés ayant 8 à 40 atomes de carbone et dérivés d'une source naturelle sont notamment les groupes alkyles dérivés de l'huile d'arachide hydrogénée, d'huile de soja et d'huile de canola (à prédominance C₁₈), l'huile de suif hydrogénée en C₁₆-C₁₈ ; et les terpénols hydrogénés en C₁₀-C₃₀, tels que le géraniol hydrogéné (en C₁₀ ramifié), le farnesol hydrogéné (C₁₅ ramifié), le phytol hydrogéné (C₂₀ ramifié).

Des exemples de phényles substitués par un alkyle en C₂-C₄₀ sont l'octylphényle, le nonylphényle, le décylphényle, le dodécylphényle, l'hexadécylphényle, l'octadécylphényle, l'isooctylphényle et le sec-butylphényle.

Des groupes alicycliques en C₈-C₄₀ peuvent être par exemple les groupes dérivés des stérols d'origine animale, tels que le cholestérol, le lanostérol, le 7-déhydrocholestérol ; ou bien les dérivés d'origine végétales, tels que le phytostérol, le stigmastérol, le campestérol ; ou bien les dérivés issus de microorganismes, tels que l'ergostérol, le mycrostérol. D'autres alicycliques en C₈-C₄₀ utilisables dans la présente invention sont par exemple le cyclooctyle, le cyclododécyle, l'adamantyle, le décahydronaphthyle, et les groupes dérivés de composés alicycliques en C₈-C₄₀ naturels tels que le pinène, le rétinol hydrogéné, le camphre et l'alcool d'isobornyle.

Les groupes alkyles en C₂-C₄₀ substitués par un groupe aryle peuvent être par exemple le 2-phényléthyle, le 2,4-diphénybutyle, le 2,4,6-triphénylhexyle, le 4-phénylbutyle, le 2-méthyl-2-phényléthyle, le 2,4,6-tri(1'phényléthyl)phényle.

A titre d'esters complexes en C₈-C₄₀ utilisables comme extrémité (iii), on peut citer l'huile de ricin hydrogénée (principalement le triglycéride de l'acide 12-hydroxystéarique ; les 1,2-diacyl glycérols tels que le 1,2-distéaryl glycérol, le 1,2-dipalmityl glycérol, le 1,2-dimyristyl glycérol ; les di-, tri- ou poly-esters de sucres tel que le 3,4,6-tristéaryl glucose, le 2,3-dilauryle fructose ; et les esters de sorbitane tels que ceux divulgués dans le brevet US 4,600,761.

Les monomères vinyliques associatifs utilisables selon l'invention peuvent être préparés par toute méthode connue dans l'art antérieur. On peut se référer par exemple aux brevets US 4,421,902, US 4,384,096, US 4,514,552, US 4,600,761, US 4,616,074, US 5,294,692, US 5,292,843 ; US 5,770,760 et US 5,412,142.

De préférence, le ou les monomères vinyliques associatifs c) utilisables selon l'invention sont choisis parmi les composés de formule (III) : 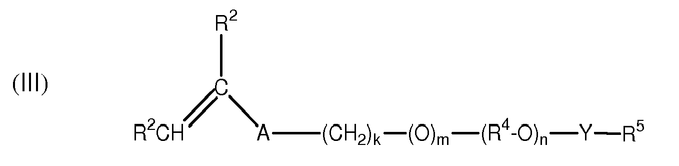 Dans laquelle
chaque R² est indépendamment H, un groupe méthyle, un groupe
-C(O)OH, ou un groupe -C(O)OR³ ;
R³ est un alkyle en C₁-C₃₀ ;
A est un groupe -CH₂C(O)O-, -C(O)O-, -O-, CH₂O, -NHC(O)NH-, -C(O)NH-, - Ar-(CE₂)_{z}-NHC(O)O-, -Ar-(CE₂)_{z} -NHC(O)NH-, ou -CH₂CH₂-NHC(O)- ;
Ar est un groupe aryle divalent ;
E est H ou un groupe méthyle ;
z est égal à 0 ou 1 ;
k est un entier variant de 0 à 30 ;
m est égal à 0 ou 1, à la condition que lorsque k = 0, m = 0, et lorsque k varie de 1 à 30, m est égal à 1 ;
(R⁴-O)ₙ est un polyoxyalkylène, qui est un homopolymère, un copolymère aléatoire, ou un copolymère à blocs, avec des unités oxyalkylène en C₂-C₄,
R⁴ est C₂H₄, C₃H₆, C₄H₈, ou leurs mélanges,
n est un entier variant de 5 à 250,
Y est -R⁴O-, -R⁴NH-, -C(O)-, -C(O)NH-, R⁴NHC(O)NH-, ou -C(O)NHC(O)-;
R⁵ est un alkyle substitué ou non choisi parmi les alkyles linéaires en C₈-C₄₀, les alkyles ramifiés en C₈-C₄₀, les alicycliques en C₈-C₄₀, les phényles substitués par un groupe alkyle en C₂-C₄₀, les alkyles en C₂-C₄₀ substitués par un groupe aryle, les esters complexes en C₈-C₈₀,
le groupe alkyle R⁵ comprenant éventuellement un ou plusieurs substituants choisis parmi les groupes hydroxyle, alcoxyle et halogèno.

De préférence, le ou les monomères vinyliques associatifs sont choisis parmi les (méth)acrylates polyéthoxylés de cétyle, les (méth)acrylates polyéthoxylés de cétéaryle, les (méth)acrylates polyéthoxylés de stéaryle, les (méth)acrylates polyéthoxylés d'arachidyle, les (méth)acrylates polyéthoxylés de béhényle, les (méth)acrylates polyéthoxylés de lauryle, les (méth)acrylates polyéthoxylés de cérotyle, les (méth)acrylates polyéthoxylés de montanyle, les (méth)acrylates polyéthoxylés de mélissyle, les (méth)acrylates polyéthoxylés de laccéryle, les (méth)acrylates polyéthoxylés de 2,4,6-tri(1'-phényléthyl)phényle, les (méth)acrylates polyéthoxylés de l'huile de ricin hydrogénée, les (méth)acrylates polyéthoxylés de canola, les (méth)acrylates polyéthoxylés du cholestérol et leurs mélanges, où la portion polyéthoxylée du monomère comprend de 5 à 100, de préférence de 10 à 80, et mieux de 15 à 60 unités oxydes d'éthylène.

Plus particulièrement, le ou les monomères vinyliques associatifs sont choisis parmi les méthacrylates polyéthoxylés de cétyle, les méthacrylates polyéthoxylés de cétéaryle, les (méth)acrylates polyéthoxylés de stéaryle, les (méth)acrylates polyéthoxylés d'arachidyle, les (méth)acrylates polyéthoxylés de béhényle, les (méth)acrylates polyéthoxylés de lauryle, où la portion polyéthoxylée du monomère comprend de 10 à 80, de préférence de 15 à 60 et mieux de 20 à 40 unités oxydes d'éthylène.

De préférence le ou les monomères associatifs vinyliques représentent de 0,001 à 25 % en poids, de préférence de 0,01 à 15 % en poids et mieux de 0,1 à 10 % en poids du mélange de monomères.

Le ou les monomères tensioactifs vinyliques semi-hydrophobes, éventuellement présents dans le mélange de monomères peuvent modérer les propriétés associatives des polymères associatifs cationiques qui les contiennent, produisant ainsi des gels aqueux ayant une très bonne texture et de très bonnes propriétés rhéologiques.

Par monomère tensioactif vinylique semi-hydrophobe, on entend au sens de la présente invention un monomère qui a une structure similaire à un monomère associatif, mais a une extrémité substantiellement non hydrophobe et ainsi ne confère pas de propriété associative aux polymères.

La propriété d'associativité d'un polymère est liée à la propriété dans un milieu donné, des molécules dudit polymère de s'associer entre elles, ou de s'associer à des molécules d'un co-agent, en général tensioactif, ce qui se traduit dans un certain domaine de concentration par un accroissement de la viscosité du milieu.

Le ou les monomères tensioactifs vinyliques semi-hydrophobes sont généralement des composés ayant deux parties :
A. un groupe terminal insaturé pour permettre la polymérisation par addition avec les autres monomères du mélange de réaction, et
B. un groupe polyoxyalkylène pour atténuer les associations entre les groupes hydrophobes du polymère ou les groupes hydrophobes des autres matériaux éventuellement présents dans la composition comprenant le polymère.

L'extrémité fournissant l'insaturation vinylique ou éthylénique pour la polymérisation par addition est de préférence dérivée d'un acide ou d'un anhydride mono ou di-carboxylique à insaturation α, β-éthylénique, de préférence un acide mono ou di-carboxylique en C₃-C₄, ou un anhydride de cet acide. De façon alternative, l'extrémité A peut dériver d'un éther allylique, d'un éther vinylique ou d'un uréthane insaturé non ionique.

L'extrémité A insaturée polymérisable peut aussi dériver d'un acide gras insaturé en C₈-C₃₀ comprenant un ou plusieurs groupe fonctionnel carboxy libre. Ce groupe en C₈-C₃₀ fait partie de l'extrémité insaturée A et est différent des groupes hydrophobes pendant des monomères associatifs, qui sont séparés de l'extrémité insaturée du monomère associatif par un groupe espaceur hydrophile.

La portion B polyoxyalkylène comprend un segment polyoxyalkylène à chaîne longue, qui est essentiellement similaire à la portion hydrophile des monomères associatifs. Des portions polyoxyalkylène B préférées incluent les unités en C₂-C₄ polyoxyéthylène, polyoxypropylène, et polyoxybutylène comprenant de 5 à 250, de préférence de 10 à 100 unités oxyalkylène. Lorsque le monomère tensioactif vinylique semi-hydrophobe comprend plus d'un type d'unité d'oxyalkylène, ces unités peuvent être disposés en séquence aléatoire, non aléatoire, ou à bloc.

De préférence, le ou les monomères tensioactifs vinyliques semi-hydrophobes sont choisis parmi les composés de formules (IV) ou (V) :

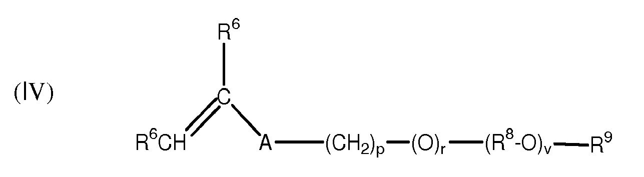

(V) D-A-(CH₂)ₚ-(O)ᵣ-(R⁸-O)ᵥ-R⁹

où, dans chaque formule (IV) ou (V),
chaque R⁶ représente indépendamment H, un alkyle en C₁-C₃₀, -C(O)OH, ou C(O)OR⁷ ;
R⁷ est un alkyle en C₁-C₃₀ ;
A est un groupe -CH₂C(O)O-, -C(O)O-, -O-, -CH₂O, -NHC(O)NH-, -C(O)NH-, -Ar-(CE₂)_{z}-NHC(O)O-, -Ar-(CE₂)_{z} -NHC(O)NH-, ou -CH₂CH₂NHC(O)- ;
Ar est un groupe aryle divalent ;
E est H ou un groupe méthyle ;
z est égal à 0 ou 1 ;
p est un entier variant de 0 à 30 ;
r est égal à 0 ou 1, à la condition que lorsque p est égal à 0, r est égal à 0, et lorsque p varie de 1 à 30, r est égal 1,
(R₈-O)ᵥ est un polyoxyalkylène qui est homopolymère, un copolymère aléatoire, ou un copolymère à blocs avec des unités oxyalkylène en C₂-C₄, où R⁸ est C₂H₄, C₃H₆, C₄H₈, ou leurs mélanges, et v est un entier variant de 5 à 250 ;
R⁹ est H ou un alkyle en C₁-C₄ ;
D est un alkyle insaturé en C₈₋C₃₀ ou un alkyle insaturé en C₈-C₃₀ substitué par un groupe carboxy.

De manière particulièrement préférée, le mélange de monomères comprend un monomère tensioactif vinylique semi-hydrophobe ayant l'une des formules suivantes :

CH₂=CH-O(CH₂)ₐO(C₃H₆O)_{b}(C₂H₄O)_{c}H

ou

CH₂=CHCH₂O(C₃H₆O)_{d}(C₂H₄O)ₑH;

où
a est égal à 2, 3, ou 4 ;
b est un entier variant de 1 à 10 ;
c est un entier variant de 5 à 50 ;
d est un entier variant de 1 à 10 ; et
e est un entier variant de 5 à 50.

Des monomères tensioactifs vinyliques semi-hydrophobes préférés sont par exemple les émulsifiants polymérisables commercialisés sous les références EMULSOGEN^{®} R109, R208, R307, RAL109, RAL208 et RAL307 par la société CLARIANT ; BX-AA-E5P5 commercialisé par la société BIMAX ; et le MAXEMUL^{®} 5010 et 5011 commercialisé par la société UNIQEMA. Les monomères particulièrement préférés sont l'EMULSOGEN^{®} R208, R307 et RAL 307.

Selon les fabricants :
l'EMULSOGEN^{®} R109 est un éther vinylique 1,4-butanediol éthoxylé/propoxylé aléatoire ayant la formule empirique :

   CH₂=CH-O(CH₂)₄O(C₃H₆O)₄(C₂H₄O)₁₀H;
l'EMULSOGEN^{®} R208 qui est un éther vinylique 1,4-butanediol éthoxylé/propoxylé aléatoire ayant la formule empirique :

   CH₂=CH-O(CH₂)₄O(C₃H₆O)₄(C₂H₄O)₂₀H;
l'EMULSOGEN^{®} R307 qui est un éther vinylique 1,4-butanediol éthoxylé/propoxylé aléatoire ayant la formule empirique :

   CH₂=CH-O(CH₂)₄O(C₃H₆O)₄(C₂H₄O)₃₀H;
l'EMULSOGEN^{®} RAL 109 qui est un éther allylique éthoxylé/propoxylé aléatoire ayant la formule empirique :

   CH₂=CHCH₂-O(C₃H₆O)₄(C₂H₄O) ₁₀H;
l'EMULSOGEN^{®} RAL 208 qui est un éther allylique éthoxylé/propoxylé aléatoire ayant la formule empirique :

   CH₂=CHCH₂-O(C₃H₆O)₄(C₂H₄O) ₂₀H;
l'EMULSOGEN^{®} RAL 307 qui est un éther allylique éthoxylé/propoxylé aléatoire ayant la formule empirique :

   CH₂=CHCH₂-O(C₃H₆O)₄(C₂H₄O) ₃₀H;
le MAXEMUL^{®} 5010 qui est un alcényle en C₁₂-C₁₅ carboxylé hydrophobe, éthoxylé avec 24 unités d'oxyde éthylène,
le MAXEMUL^{®} 5011 qui est un alcényle en C₁₂-C₁₅ carboxylé hydrophobe, éthoxylé avec 34 unités d'oxyde éthylène ;
et le BX-AA-E5P5 qui est un éther allylique éthoxylé/propoxylé aléatoire ayant la formule empirique :

   CH₂₌CHCH₂-O(C₃H₆O)₅(C₂H₄O) ₅H.

La quantité du ou des monomères tensioactifs vinyliques semi-hydrophobes utilisés dans la préparation des polymères cationiques (i) utilisés dans la composition selon l'invention peut varier largement et dépend, en autre, des propriétés rhéologiques finales désirées pour le polymère.

Lorsqu'ils sont présents, les monomères tensioactifs vinyliques semi-hydrophobes représentent de 0,01 à 25 % en poids et de préférence de 0,1 à 10% en poids par rapport au poids total du mélange de monomères.

Le ou les polymères cationiques (i) utilisés dans la composition selon l'invention sont préparés à partir d'un mélange de monomères pouvant comprendre un ou plusieurs monomères vinyliques non ioniques hydroxylés.

Ces monomères sont des monomères à insaturation(s) éthylénique(s) comprenant un ou plusieurs substituants hydroxyle.

A titre de monomères vinyliques non ioniques hydroxylés, on peut citer les (méth)acrylates d'alkyle en C₁-C₆ hydroxylés, de préférence les (méth)acrylates d'alkyle en C₁-C₄ hydroxylés, tel que le 2-hydroxyéthylméthacrylate (HEMA), le 2-hydroxyéthyl acrylate (2-HEA), le 3-hydroxypropyl acrylate ; les (méth)acrylamides d'alkyle en C₁-C₄ hydroxylés, tels que le N-(2-hydroxyéthyl) méthacrylamide, le N-(2-hydroxyéthyl) acrylamide, le N-(3-hydroxypropyl) acrylamide, le N-(2,3-dihydroxypropyl) acrylamide ; et leurs mélanges. On peut encore citer l'alcool allylique, le glycérol monoallyl éther, le 3-méthyl-3-butèn-1-ol, les précurseurs de l'alcool vinylique et leurs équivalents, tel que l'acétate de vinyle.

Lorsqu'il(s) est (sont) présent(s), le ou les monomères vinyliques non ioniques hydroxylés représentent généralement jusqu'à 10% en poids du poids total du mélange de monomères. De préférence, le ou les monomères vinyliques non ioniques hydroxylés représentent de 0,01 à 10% en poids, mieux de 1 à 8%, et encore de 1 à 5% en poids par rapport au poids total du mélange de monomères.

Le ou les polymères cationiques utilisés (i) dans la composition selon l'invention sont préparés à partir d'un mélange de monomères qui peut comprendre un ou plusieurs monomères réticulants permettant d'introduire des ramifications et de contrôler la masse moléculaire.

Des agents réticulants poly-insaturés utilisables sont bien connus dans l'état de la technique. Des composés mono-insaturés présentant un groupe réactif capable de réticuler un copolymère formé avant, pendant ou après la polymérisation peuvent aussi être utilisés. D'autres monomères réticulant utilisables peuvent être des monomères polyfonctionnels comprenant des groupes réactifs multiples tels que des groupes époxydes, isocyanates et des groupes silanes hydrolysables. De nombreux composés poly-insaturés peuvent être utilisés pour générer un réseau tri-dimensionnel partiellement ou substantiellement réticulé.

Des exemples de monomères réticulants polyinsaturés utilisables sont par exemple les monomères aromatiques polyinsaturés, tel que le divinylbenzène, le divinyl naphtylène, et le trivinylbenzène ; les monomères alicycliques polyinsaturés, tel que le 1,2,4-trivinylcyclohexane ; les esters difonctionnels de l'acide phthalique, tel que le diallyl phthalate ; les monomères aliphatiques polyinsaturés, tels que les diènes, les triènes et les tétraènes, notamment l'isoprène, le butadiène, le 1,5-hexadiène, le 1,5,9-décatriène, le 1,9-décadiène, et le 1,5 heptadiène.

D'autres monomères réticulants polyinsaturés utilisables sont par exemple les éthers polyalcényles, tels que le triallyl pentaérythritol, le diallyl pentaérythritol, le diallyl saccharose, l'octaallyl saccharose et le triméthylolpropane diallyl éther ; les esters polyinsaturés de polyalcools ou de polyacides, tel que le 1,6-hexanediol di(méth)acrylate, le tétraméthylène tri(méth)acrylate, l'allyl acrylate, le diallyl itaconate, le diallyl fumarate, le diallyl maléate, le triméthylolpropane tri(méth)acrylate, le triméthylolpropane di(méth)acrylate et le polyéthylène glycol di(méth)acrylate, les alkylène bisacrylamides, tel que le méthylène bisacrylamide, le propylène bisacrylamide ; les dérivés hydroxylés et carboxylés du méthylène bisacrylamide, tel que le N,N'-bisméthylol méthylène bisacrylamide ; les polyéthylèneglycol di(méth)acrylates, tels que l'éthylèneglycol di(méth)acrylate, le diiéthylèneglycol di(méth)acrylate, le triéthylèneglycol di(méth)acrylate, les silanes polyinsaturés tels que le diméthyldivinylsilane, le méthyltrivinylsilane, l'allyldiméthylvinylsilane, le diallyldiméthylsilane et le tétravinylsilane, les stannanes polyinsaturés, tel que le tétraallyl étain et le diallyldiméthyl étain.

Des monomères réticulants monoinsaturés utilisables et portant un groupe réactif peuvent être les N-méthylolacrylamides ; les N-alkoxy(méth)acrylamides, où le groupe alkoxy est un groupe C₁-C₁₈ ; et les silanes hydrolysables insaturés tel que triéthoxyvinylsilane, le tris-isopropoxyvinylsilane, et le 3-triéthoxysilylpropyl méthacrylate.

Des monomères réticulants polyfonctionnels utilisables et comprenant plusieurs groupes réactifs peuvent être par exemple les silanes hydrolysables tel que l'éthyltriéthoxysilane et l'éthyltriméthoxysilane; les silanes hydrolysables époxylés, tel que le 2-(3,4-époxycyclohexyl)éthyltriéthoxysilane et le 3-glycidoxypropyltriméthyoxysilane; les polyisocyanates, tels que le 1,4-diisocyanatobutane, le 1,6-diisocyanatohexane, le 1,4-phénylènediisocyanate, et le 4,4'-oxybis(phénylisocyanate) ; les époxides insaturés, tel que le glycidyl méthacrylate et l'allylglycidyl éther ; les polyépoxides, tel que le diglycidyl éther, le 1,2,5,6-diépoxyhexane, et l'éthylèneglycoldiglycidyl éther.

Des monomères réticulants polyinsaturés particulièrement utilisables sont les polyols éthoxylés, tels les diols, les triols et les bis-phénols, éthoxylés avec 2 à 100 moles d'oxyde d'éthylène par mole de groupe fonctionnel hydroxyle et terminés par un groupe insaturé polymérisable tel que un vinyl éther, un allyl éther, un ester acrylate ou un ester méthacrylate. De tels monomères réticulants peuvent être par exemple le diméthacrylate éthoxylé de biphénol A, le diméthacrylate éthoxylé de biphénol F, et le triméthylol propane triméthacrylate éthoxylé.

D'autres monomères réticulants éthoxylés utilisables dans la présente invention sont par exemple les agents réticulants dérivés des polyols éthoxylés divulgués dans le brevet US 6 140 435.

Des exemples particulièrement préférés de monomères réticulants sont des esters acrylates et méthacrylates de polyols ayant au moins deux groupes ester acrylate ou méthacrylate, tel que le triméthylolpropane triacrylate (TMPTA), le triméthylolpropane diméthacrylate, le triéthylène glycol diméthacrylate (TEGDMA), et le diméthacrylate de bisphénol A éthoxylé (30) (EOBDMA).

Lorsqu'il(s) est (sont) présent(s), le ou les monomères réticulants représentent de préférence au plus 5% en poids par rapport au poids du mélange de monomères. Selon un mode de réalisation préféré, les monomères réticulants sont présents à une teneur variant de 0,001 à 5% en poids, de préférence de 0,05 à 2% en poids, mieux de 0,1 à 1% en poids par rapport au poids total du mélange de monomères.

Le mélange de monomères peut comprendre un ou plusieurs agents de transfert de chaîne. Les agents de transferts de chaîne sont des composés bien connus de l'état de la technique.

On peut citer en particulier les composés thiolés, les composés disulfures, tels que les C₁-C₁₈ mercaptans, les acides mercaptocarboxyliques, les esters d'acides mercaptocarboxyliques, les thioesters, les C₁-C₁₈ alkyl disulfures, les aryldisulfures, les thiols polyfonctionnels ; les phosphites et hypophosphites ; les composés haloalkyl, tel que le tétrachlorure de carbone, le bromotrichlorométhane ; et les agents de transfert de chaîne insaturés, tel que l'alphaméthylstyrène.

Les thiols polyfonctionnels sont par exemple les thiols trifonctionnels, tel que le triméthylolpropane-tris-(3-mercaptopropionate), les thiols tétrafonctionnels, tel que le pentaérythritol-tétra-(3-mercaptopropionate), le pentaérythritol-tétra-(thioglycolate) et le pentaérythritol-tétra(thiolactate) ; les thiols hexafonctionnels, tel que le pentaérytritol-hexa-(thioglyconate).

De façon alternative, le ou les agents de transfert de chaîne peuvent être des agents de transfert de chaîne catalytiques qui réduisent le poids moléculaire des polymères d'addition lors de la polymérisation par radicaux libres des monomères vinyliques. On peut citer par exemple les complexes de cobalt, notamment les chélates de cobalt (II). Les agents de transfert de chaîne catalytiques peuvent souvent être utilisés à des concentrations faibles par rapport aux agents de transfert de chaîne thiolés.

De façon particulièrement préférée, on peut citer à titre d'agent de transfert de chaîne l'octyl mercaptan, le n-dodécyle mercaptan, le t-dodécyle mercaptan, l'hexadécyle mercaptan, l'octadécyle mercaptan (ODM), l'isooctyl 3-mercaptopropionate (IMP), le butyl 3-mercaptopropionate, l'acide 3-mercaptopropionique, le butyl thioglycolate, l'isooctyl thioglycolate, le dodécyle thioglycolate.

Lorsqu'il(s) est (sont) présents, le ou les agents de transferts de chaîne sont ajoutés au mélange de monomères de préférence jusqu'à 10% en poids par rapport au poids total du mélange de monomère. De préférence, le ou les agents de transfert de chaîne représentent de 0,1% à 5 % en poids par rapport au poids total de monomères.

Le mélange de monomères permettant la préparation du polymère cationique (i) utilisé dans la composition selon l'invention peut comprendre un ou plusieurs stabilisateurs polymériques pour l'obtention de dispersions ou d'émulsions stables. De préférence, les polymères sont solubles dans l'eau. On peut citer par exemple les polymères synthétiques, tels que les alcools polyvinyliques, les acétates polyvinyliques partiellement hydrolysés, la polyvinylpyrrolidone, les polyacrylamides, les polyméthacrylamides, les polymères d'addition carboxylés, les polyalkyles vinyle éthers ; les polymères naturels hydrosolubles, tels que la gélatine, les peptines, les alginates, la caséine; les polymères naturels modifiés, tels que la méthylcellulose, l'hydroxypropylcellulose, la carboxyméthylcellulose, les hydroxyéthylcelluloses allyliques.

Les stabilisateurs polymériques sont utilisés en une quantité au plus égale à 2 % en poids par rapport au poids total de l'émulsion, de préférence en une quantité comprise 0,0001 et 1 % en poids, mieux entre 0,01 et 0,5 % en poids par rapport au poids du mélange de monomères.

Selon un mode de réalisation préféré, le mélange de monomères comprend, par rapport au poids total du mélange de monomères :
a) de 10 à 70 % en poids du ou des monomères vinyliques substitués par un ou plusieurs groupes amino,
b) de 20 à 80 % en poids du ou des monomères vinyliques non ioniques hydrophobes,
c) de 0,001 à 25 % en poids du ou des monomères vinyliques associatifs,
d) de 0 à 25 % en poids du ou des monomères tensioactifs vinyliques semi-hydrophobes,
e) de 0 à 10 % en poids d'un ou plusieurs monomères vinyliques non ioniques hydroxylés,
f) de 0 à 5% en poids d'un ou plusieurs monomères réticulants,
g) de 0 à 10 % en poids d'un ou plusieurs agents de transfert de chaîne, et
h) de 0 à 2 % en poids d'un ou plusieurs stabilisateurs polymériques.

De façon encore plus préférée, le mélange de monomères comprend, par rapport au poids total du mélange de monomères :
a) de 20 à 60 % en poids du ou des monomères vinyliques substitués par un ou plusieurs groupes amino,
b) de 20 à 70 % en poids du ou des monomères vinyliques non ioniques hydrophobes,
c) de 0,01 à 15 % en poids du ou des monomères vinyliques associatifs,
d) de 0,1 à 10 % en poids du ou des monomères tensioactifs vinyliques semi-hydrophobes,
e) de 0,01 à 10 % en poids du ou des monomères vinyliques non ioniques hydroxylés,
f) de 0,001 à 5 % en poids du ou des monomères réticulants, et
g) de 0,001 à 10 % en poids du ou des agents de transfert de chaîne,
h) de 0 à 2 % en poids du ou des stabilisateurs polymériques.

Selon un mode de réalisation particulièrement préféré, le mélange de monomères permettant la préparation du polymère cationique (i) utilisé dans la composition selon l'invention comprend, par rapport au poids total du mélange de monomères :
a) de 20 à 50 % en poids d'un ou plusieurs monomères vinyliques substitués par un ou plusieurs groupes amino choisis parmi :
   - le (méth)acrylate de 3-(N,N-diméthylamino)propyle,
   - le N'-(3-N,N-diméthylamino)propyl(méth)acrylamide,
   - le (méth)acrylate de 2-(N,N-diméthylamino)éthyle,
   - le (méth)acrylate de 2-(N,N-diéthylamino)éthyle,
   - le (méth)acrylate de 2-(tert-butylamino)éthyle,
   - le 2-(N,N-diméthylamino)propyl (méth)acrylamide, et
   - l'acrylate de 2-(N,N-diméthylamino)néopentyle,
b) de 50 à 65 % en poids d'un ou plusieurs monomères vinyliques non ioniques hydrophobes choisis parmi les esters d'acide acrylique et d'alkyle en C₁-C₃₀, les esters de l'acide méthacrylique et d'alkyle en C₁-C₃₀, et leurs mélanges,
c) de 0,1 à 10 % en poids d'un ou plusieurs monomères vinyliques associatifs choisis parmi les méthacrylates polyéthoxylés de cétyle, les méthacrylates polyéthoxylés de cétéaryle, les (méth)acrylates polyéthoxylés de stéaryle, les (méth)acrylates polyéthoxylés d'arachidyle, les (méth)acrylates polyéthoxylés de béhényle, les (méth)acrylates polyéthoxylés de lauryle, les (méth)acrylates polyéthoxylés de cérotyle, les (méth)acrylates polyéthoxylés de montanyle, les (méth)acrylates polyéthoxylés de mélissyle, les (méth)acrylates polyéthoxylés de laccéryle, les (méth)acrylates polyéthoxylés de 2,4,6 (1'-phényléthyl)phényle, les (méth)acrylates polyéthoxylés de l'huile de ricin hydrogénée, les (méth)acrylates polyéthoxylés de canola, les (méth)acrylates polyéthoxylés du cholestérol et leurs mélanges,
d) de 0,1 à 10 % en poids d'un ou plusieurs monomères tensioactifs vinyliques semi-hydrophobes ayant l'une des formules suivantes :

   CH₂₌CH-O(CH₂)ₐO(C₃H₆O)_{b}(C₂H₄O)_{c}H ou

   CH₂=CHCH₂O(C₃H₆O)_{d}(C₂H₄O)ₑH;

   où
   a est égal à 2, 3, ou 4 ;
   b est un entier variant de 1 à 10 ;
   c est un entier variant de 5 à 50 ;
   d est un entier variant de 1 à 10 ; et
   e est un entier variant de 5 à 50.
e) jusqu'à 10 % en poids d'un ou plusieurs monomères vinyliques non ioniques hydroxylés,
f) jusqu'à 5% en poids d'un ou plusieurs monomères réticulants,
g) jusqu'à 10 % en poids d'un ou plusieurs agents de transfert de chaîne, et
h) jusqu'à 2 % en poids d'un ou plusieurs stabilisateurs polymériques.

Les polymères cationiques (i) préférés selon l'invention sont des polymères issus de la polymérisation du mélange de monomères suivants :
- un méthacrylate de di(alkyl en C₁-C₄)amino(alkyle en C₁-C₆),
- un ou plusieurs esters d'alkyle en C₁-C₃₀ et de l'acide (méth)acrylique,
- un méthacrylate d'alkyle en C₁₀-C₃₀ polyéthoxylé avec 20 à 30 moles d'oxyde d'éthylène,
- un allyl éther de polyéthylèneglycol/polypropylèneglycol 30/5,
- un méthacrylate d'hydroxy(alkyle en C₂-C₆),
- un diméthacrylate d'éthylèneglycol.

Parmi les polymères cationiques (i) utilisés dans la composition selon l'invention, on peut notamment citer le composé commercialisé par la société NOVEON sous la dénomination CARBOPOL AQUA CC POLYMER et qui correspond à la dénomination INCl POLYACRYLATE-1 CROSSPOLYMER.

Le POLYACRYLATE-1 CROSSPOLYMER est le produit de la polymérisation d'un mélange de monomères comprenant (ou constitué de):
- un méthacrylate de di(alkyl en C₁-C₄) amino(alkyle en C₁-C₆),
- un ou plusieurs esters d'alkyle en C₁-C₃₀ et de l'acide (méth)acrylique,
- un méthacrylate d'alkyle en C₁₀-C₃₀ polyéthoxylé (20-25 moles de motif oxyde d'éthylène),
- un allyl éther de polyéthylèneglycol/polypropylèneglycol 30/5,
- un méthacrylate d'hydroxy(alkyle en C₂-C₆), et
- un diméthacrylate d'éthylèneglycol.

Le ou les polymères cationiques (i) utilisés dans les compositions selon l'invention représentent généralement de 0,01 à 10% en poids, de préférence de 0,05 à 5% en poids, et mieux de 0,1 à 1% en poids par rapport au poids total de la composition.

Le ou les polymères cationiques (i) utilisés dans la composition selon l'invention peuvent être préparés par des techniques de polymérisation conventionnelles, telles que la polymérisation en émulsion, comme cela est bien connu dans le domaine des polymères. La polymérisation peut être effectuée par simple procédé discontinu, par procédé par addition contrôlée, ou bien la réaction peut être initiée dans un petit réacteur puis alors la masse des monomères peut être ajoutée de façon contrôlée dans le réacteur (procédé par ensemencement). Généralement, la polymérisation est menée à une température de réaction comprise entre 20 et 80°C, même si des températures supérieures ou inférieures peuvent être utilisées. Pour faciliter l'émulsification du mélange de monomères, la polymérisation par émulsion est effectuée en présence d'un tensioactif présent en une quantité variant de 1 à 10 % en poids, de préférence de 3 à 8% en poids, mieux de 5 à 7 % en poids, par rapport au poids total de l'émulsion. Le milieu de réaction de polymérisation par émulsion comprend également un ou plusieurs initiateurs radicalaires, de préférence en une quantité variante de 0,01 à 3 % en poids par rapport au poids total du mélange de monomères. La polymérisation peut être réalisée dans un milieu aqueux ou bien hydroalcoolique à un pH neutre ou faiblement alcalin.

Dans une polymérisation typique, le mélange de monomères est ajouté sous agitation à une solution de tensioactifs émulsifiants, tel qu'un tensioactif non ionique, de préférence un éthoxylate d'alcool linéaire ou ramifié, ou un mélange de tensioactifs non ioniques et anioniques, tels que des sulfates d'alcool gras ou des alkyles sulfonates d'alcool gras, dans une quantité adaptée d'eau, dans un réacteur adapté, pour préparer l'émulsion de monomères. L'émulsion est désoxygénée au moyen de toute méthode connue, puis la réaction de polymérisation est initiée en ajoutant un catalyseur de polymérisation (initiateur) tel que le persulfate de sodium, ou tout autre catalyseur de polymérisation par addition adaptée, comme cela est bien connu dans le domaine des polymères. La réaction est agitée jusqu'à ce que la polymérisation soit complète, généralement pendant une durée variant de 4 heures à 16 heures. L'émulsion de monomères peut être chauffée à une température comprise entre 20 et 80°C avant l'addition de l'initiateur, si cela est souhaité. La quantité de monomères n'ayant pas réagi peut être éliminée par addition d'une quantité supplémentaire de catalyseur. L'émulsion de polymère obtenue peut être retirée du réacteur et emballée pour être stockée ou utilisée. De façon optionnelle, le pH ou d'autres caractéristiques physiques ou chimiques de l'émulsion peuvent être ajustés avant de retirer l'émulsion du réacteur. Généralement, l'émulsion produite a une teneur totale en solides qui varie entre 10 et 40 % en poids. Généralement, la quantité totale de polymères dans l'émulsion obtenue varie entre 15 et 35% en poids, en général au plus 25 % en poids.

Des tensioactifs adaptés pour faciliter la polymérisation par émulsion peuvent être des tensioactifs non ioniques, anioniques, amphotères ou cationiques, ou leurs mélanges. Le plus souvent, des tensioactifs non ioniques ou anioniques, ou leurs mélanges sont utilisés.

Tous types de tensioactifs non ioniques, anioniques, amphotères ou cationiques classiquement utilisés dans les polymérisations en l'émulsion peuvent être utilisés.

La polymérisation peut être effectuée en présence d'un ou plusieurs initiateurs de radicaux libres. Ceux-ci peuvent être choisis parmi les composés persulfates inorganiques insolubles, tels que le persulfate d'ammonium, le persulfate de potassium, le persulfate de sodium ; les peroxydes tels que le peroxyde d'hydrogène, le peroxyde de benzoyle, le peroxyde d'acétyle, et le peroxyde de lauryle ; les hydroperoxydes organiques, tels que l'hydroperoxyde de cumen et l'hydroperoxyde de t-butyle ; les peracides organiques, tel que l'acide peracétique ; et les agents producteurs de radicaux libres solubles dans l'huile, tels que 2,2'-azobisisobutyronitrile, et leurs mélanges. Les peroxydes et les peracides peuvent être éventuellement activés avec des agents réducteurs, tel que le bisulfite de sodium ou l'acide ascorbique, les métaux de transition, l'hydrazine. Des initiateurs de radicaux libres particulièrement adaptés sont les initiateurs de polymérisation azoïque solubles dans l'eau, tels que les composés 2,2'-azobis(tert-alkyl) ayant un substituant hydrosolubilisant sur le groupe alkyle. Des catalyseurs de polymérisation azoïque préférés sont les initiateurs de radicaux libres VAZO^{®}, commercialisés par la société DuPont, tel que le VAZO^{®}44 (2,2'-azobis(2-4,5-dihydroimidazolyl)propane), le VAZO^{®}56 (2,2'-azobis(2-méthylpropio-namidine)dihydrochlorure), et le VAZO^{®}68 (4,4'-azobis(acide 4-cyanovalérique)).

Les amidons utilisables dans la présente invention sont plus particulièrement des macromolécules sous forme de polymères constitués de motifs élémentaires qui sont des unités anhydroglucose. Le nombre de ces motifs et leur assemblage permettent de distinguer l'amylose (polymère linéaire) et l'amylopectine (polymère ramifié). Les proportions relatives d'amylose et d'amylopectine, ainsi que leur degré de polymérisation, varient en fonction de l'origine botanique des amidons.

Les molécules d'amidons utilisés dans la présente invention peuvent avoir comme origine botanique les céréales ou encore les tubercules. Ainsi, les amidons sont par exemple choisis parmi les amidons de maïs, de riz, de manioc, d'orge, de pomme de terre, de blé, de sorgho, de pois.

Les amidons utilisés dans la composition de l'invention sont de préférence modifiés. Les amidons peuvent être modifiés par voie chimique ou physique : notamment par une ou plusieurs des réactions suivantes : prégélatinisation, oxydation, réticulation, estérification, éthérification, amidification, traitements thermiques.

De manière plus particulière, ces réactions peuvent être réalisées de la façon suivante :
- prégélatinisation en faisant éclater les granules d'amidon (par exemple séchage et cuisson dans un tambour sécheur) ;
- oxydation par des oxydants forts conduisant à l'introduction de groupes carboxyle dans la molécule d'amidon et à la dépolymèrisation de la molécule d'amidon (par exemple en traitant une solution aqueuse d'amidon par l'hypochlorite de sodium) ;
- réticulation par des agents fonctionnels capables de réagir avec les groupes hydroxyle des molécules d'amidon qui vont ainsi être liées entre elles (par exemple avec des groupes glyceryl et/ou phosphate) ;
- estérification en milieu alcalin pour le greffage de groupes fonctionnels, notamment acyl en en C1-C6 (acétyl), hydroxyalkylés en C1-C6 (hydroxyéthyl, hydroxypropyl), carboxyméthyl, octénylsuccinique.

On peut notamment obtenir par réticulation avec des composés phophorés des phosphates de monoamidon ( du type Am-O-PO-(OX)₂), des phosphates de diamidon ( du type Am-O-PO-(OX)-O-Am) ou même de triamidon (du type Am-O-PO- (O-Am)₂) ou leurs mélanges.

X désigne notamment les métaux alcalins (par exemple sodium ou potassium), les métaux alcalinoterreux (par exemple calcium, magnésium), les sels d'ammoniaque, les sels d'amines comme ceux de la monoéthanolamine, la diéthanolamine, la triéthanolamine, l'amino-3 propanediol-1,2, les sels ammoniums issus des aminoacides basiques tels que la lysine, l'arginine, la sarcosine, l'ornithine, la citrulline.

Les composés phosphorés peuvent être par exemple du tripolyphosphate de sodium, de l'orthophosphate de sodium, de l'oxychlorure de phosphore ou du trimétaphosphate de sodium.

On utilisera préférentiellement des phosphates de diamidon ou des composés riches en phosphate de diamidon comme le produit proposé sous les références PREJEL VA-70-T AGGL (phosphate de diamidon de manioc hydroxypropylé gélatinisé) ou PREJEL TK1 (phosphate de diamidon de manioc gélatinisé) ou PREJEL 200 (phosphate de diamidon de manioc acétylé gélatinisé) par la Société AVEBE ou STRUCTURE ZEA de NATIONAL STARCH (phosphate de diamidon de maïs gélatinisé).

Un amidon préféré est un amidon ayant subi au moins une modification voie chimique telle qu'au moins une estérification.

Selon l'invention, on peut aussi utiliser des amidons amphotères, ces amidons amphotères comprennent un ou plusieurs groupements anioniques et un ou plusieurs groupements cationiques. Les groupements anioniques et cationiques peuvent être liés au même site réactif de la molécule d'amidon ou à des sites réactifs différents; de préférence ils sont liés au même site réactif. Les groupements anioniques peuvent être de type carboxylique, phosphate ou sulfate et de préférence carboxylique. Les groupements cationiques peuvent être de type amine primaire, secondaire, tertiaire ou quaternaire.

Les amidons amphotères sont notamment choisis parmi les composés de formules suivantes : 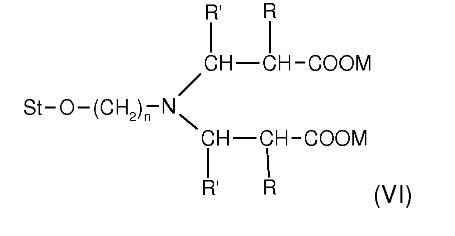 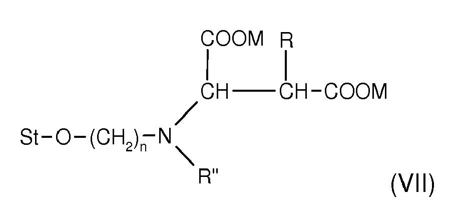 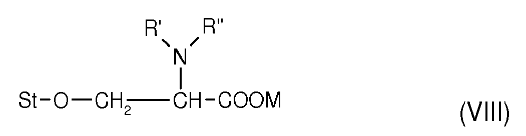 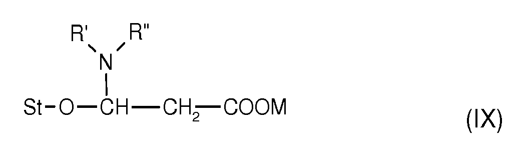 formules dans lesquelles :
St-O représente une molécule d'amidon,
R, identique ou différent, représente un atome d'hydrogène ou un radical méthyle,
R', identique ou différent, représente un atome d'hydrogène, un radical méthyle ou un groupement -COOH,
n est un entier égal à 2 ou 3,
M, identique ou différent, désigne un atome d'hydrogène, un métal alcalin ou alcalinoterreux tels que Na, K, Li, NH₄ , un ammonium quaternaire ou une amine organique,
R" représente un atome d'hydrogène ou un radical alkyle ayant de 1 à 18 atomes de carbone.

Ces composés sont notamment décrits dans les brevets US 5,455,340 et US 4,017,460 qui sont inclus à titre de référence.

Les molécules d'amidons peuvent être issues de toutes les sources végétales d'amidon telles que notamment le maïs, la pomme de terre, l'avoine, le riz, le tapioca, le sorgho, l'orge ou le blé. On peut également utiliser les hydrolysats des amidons cités ci-dessus. L'amidon est de préférence issu de la pomme de terre.

On utilise particulièrement les amidons de formules (VI) ou (VII). On utilise plus particulièrement les amidons modifiés par de l'acide 2-chloroéthyl aminodipropionique, c'est à dire les amidons de formule (VI) ou (VII) dans lesquelles R, R', R" et M représentent un atome d'hydrogène et n est égal à 2. L'amidon amphotère préféré est un chloroéthylamidodipropionate d'amidon.

Selon l'invention, le ou les amidons peuvent représenter de 0,1 % à 20 % en poids, de préférence de 0,5 % à 15% en poids et plus particulièrement de 1 à 10% en poids par rapport au poids total de la composition finale.

De préférence, le rapport pondéral entre le ou les polymères cationiques (i) décrits ci-dessus et le ou les amidons va de 0,01 à 10, plus particulièrement de 0,05 à 5, mieux de 0,1 à 1.

Les compositions selon l'invention contiennent de préférence en outre un ou plusieurs agents tensio-actifs, notamment choisis parmi les tensio-actifs anioniques, amphotères, non-ioniques, cationiques et leurs mélanges.

Parmi les agents tensio-actifs anioniques, on peut citer les sels alcalins, les sels d'ammonium, les sels d'amines, les sels d'aminoalcools, les sels de magnésium des composés suivants : les alkylsulfates, alkyléthersulfates, alkylamidoéthersulfates, alkylarylpolyéthersulfates, monoglycérides sulfates; les alkylsulfonates, alkylamides sulfonates, alkylarylsulfonates, oléfines sulfonates, paraffines sulfonates; les alkylsulfosuccinates, les alkyléthersulfosuccinates, les alkylamides sulfosuccinates; les alkylsulfosuccinamates; les alkylsulfoacétates; les alkylphosphates, alkyléther phosphates; les acylsarcosinates, les acyliséthionates et les N-acyltaurates.

Le radical alkyle ou acyle de ces différents composés est généralement constitué par une chaîne carbonée comportant de 8 à 30 atomes de carbone.

Parmi les agents tensio-actifs anioniques, on peut également citer les sels d'acides gras tels que les sels des acides oléique, ricinoléique, palmitique, stéarique; les acides d'huile de coprah ou d'huile de coprah hydrogénée; les acyl lactylates, dont le radical acyle comporte de 8 à 30 atomes de carbone.

On peut également utiliser des agents tensio-actifs considérés comme faiblement anioniques tels que les acides alkyl ou alkylaryl éthers carboxyliques polyoxyalkylénés ou leurs sels, les acides alkylamido éthers carboxyliques polyoxyalkylénés ou leurs sels, les acides d'alkyl D-galactoside uroniques ou leurs sels.

Les agents tensio-actifs non-ioniques sont plus particulièrement choisis parmi les alcools ou les alkylphénols ou les acides gras polyéthoxylés, polypropoxylés ou polyglycérolés, à chaîne grasse comportant 8 à 30 atomes de carbone, le nombre de groupements oxyde d'éthylène ou oxyde de propylène étant compris entre 2 et 50 et le nombre de groupements glycérol étant compris entre 2 et 30.

On peut également citer les copolymères d'oxydes d'éthylène et de propylène; les condensats d'oxydes d'éthylène et de propylène sur des alcools gras; les amides gras polyéthoxylés ayant de préférence 2 à 30 moles d'oxyde d'éthylène; les amides gras polyglycérolés comportant de préférence 1 à 5 groupements glycérol et en particulier 1,5 à 4; les amines grasses polyéthoxylées ayant de préférence 2 à 30 moles d'oxyde d'éthylène; les esters d'acides gras du sorbitan oxyéthylénés avec 2 à 30 moles d'oxyde d'éthylène; les esters d'acide gras de sucrose, les esters d'acides gras du polyéthylèneglycol, les alkylpolyglycosides, les dérivés carbamates ou amides de N-alkyl glucamines, les aldobionamides, les oxydes d'amines tels que les oxydes d'alkylamines ou de N-acylamidopropyl-morpholine.

Les agents tensio-actifs amphotères préférés sont les dérivés d'amines secondaires ou tertiaires aliphatiques, dans lesquels le radical aliphatique est une chaîne linéaire ou ramifiée comportant 8 à 22 atomes de carbone et qui contient au moins un groupe anionique hydrosolubilisant, carboxylate, sulfonate, sulfate, phosphate ou phosphonate; les alkyl(C₈-C₂₀)bétaïnes, les sulfobétaïnes, les alkyl(C₈-C₂₀)amidoalkyl(C₁-C₆) bétaïnes ou les alkyl(C₈-C₂₀)amidoalkyl(C₁-C₆)sulfobétaïnes.

Parmi les dérivés d'amines, on peut citer les produits vendus sous la dénomination MIRANOL, tels que décrits dans les brevets US-A-2 528 378 et 2 781 354 et classés dans le dictionnaire CTFA, 7ème édition, 1997, sous la dénomination Disodium Cocoamphodiacétate, Disodium Lauroamphodiacétate, Disodium Capryloamphodiacétate, Disodium Caproamphodiacétate, Disodium Cocoamphodipropionate, Disodium Lauroamphodipropionate, Disodium Caproampho-dipropionate, Disodium Capryloamphodipropionate, Lauroampho-dipropionate acide, Cocoamphodipropionate acide.

Les agents tensio-actifs cationiques sont notamment choisis parmi les sels d'amines grasses primaires, secondaires ou tertiaires, éventuellement polyoxyalkylénées; les sels d'ammonium quaternaire; les dérivés d'imidazoline; ou les oxydes d'amines à caractère cationique.

Les sels d'ammonium quaternaire préférés sont les halogénures (par ex. chlorures) de tétraalkylammonium comme par exemple les chlorures de dialkyldiméthylammonium ou d'alkyltriméthylammonium, dans lesquels le radical alkyl comporte environ de 12 à 22 atomes de carbone, en particulier les chlorures de béhényltriméthylammonium, de distéaryldiméthylammonium, de cétyltriméthylammonium, de benzyl diméthyl stéaryl ammonium ou encore le chlorure de stéaramidopropyldiméthyl (myristyl acétate) ammonium commercialisé sous la dénomination «CEPHARYL 70» par la société VAN DYK.

On peut également utiliser les sels (chlorures ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme ou de tournesol.

Les agents tensio-actifs sont éventuellement utilisés dans les compositions conformes à l'invention dans des proportions de 0,01 à 50% en poids par rapport au poids total de la composition. Quand les compositions sont sous forme de shampooings, ils sont généralement utilisés à raison d'au moins 4% en poids, de préférence entre 5 et 50% en poids par rapport au poids total de la composition, et en particulier entre 8 et 35%.

Les compositions, selon l'invention, présentent un pH généralement compris entre 3 et 12, et plus particulièrement entre 4 et 8 et de préférence entre 4 et 6 (bornes comprises).

Selon une variante préférée de l'invention, les compositions cosmétiques peuvent également contenir des agents de conditionnement des matières kératiniques.

Lorsque la composition contient au moins un agent de conditionnement, ils sont généralement choisis parmi les huiles de synthèse telles que les poly-α-oléfines, les huiles fluorées, les cires fluorées, les gommes fluorées, les esters d'acides carboxyliques insolubles dans l'eau, les polymères cationiques autres que les polymères (i) de l'invention, les silicones, les huiles minérales, végétales ou animales, les céramides, les pseudocéramides et leurs mélanges.

Les polyoléfines sont de préférence des poly-α-oléfines et en particulier :
- de type polybutène, hydrogéné ou non, et de préférence polyisobutène, hydrogéné ou non.

On utilise de préférence les oligomères d'isobutylène de poids moléculaire inférieur à 1000 et leurs mélange avec des polyisobutylènes de poids moléculaire supérieur à 1000 et de préférence allant de 1000 à 15000.

A titre d'exemples de poly-α-oléfines utilisables dans le cadre de la présente invention, on peut plus particulièrement mentionner les polyisobutènes vendus sous le nom de PERMETHYL 99 A, 101 A , 102 A , 104 A (n=16) et 106 A (n=38) par la Société PRESPERSE Inc, ou bien encore les produits vendus sous le nom de ARLAMOL HD (n=3) par la Société ICI (n désignant le degré de polymérisation),
- de type polydécène, hydrogéné ou non.

De tels produits sont vendus par exemple sous les dénominations ETHYLFLO par la société ETHYL CORP., et d'ARLAMOL PAO par la société ICI.

Les huiles minérales pouvant être utilisées dans les compositions de l'invention sont choisies préférentiellement dans le groupe formé par :
- les hydrocarbures, tels que l'hexadécane et l'huile de paraffine ;

Dans un autre mode de réalisation préféré, les compositions de l'invention comprennent en outre un ou plusieurs polymères cationiques différents des polymères cationiques (i) de l'invention choisis parmi tous ceux déjà connus en soi, notamment ceux décrits dans la demande de brevet EP-A-0 337 354 et dans les demandes de brevets français FR-A-2 270 846, 2 383 660, 2 598 611, 2 470 596 et 2 519 863.

Les polymères cationiques utilisés ont généralement une masse moléculaire comprise entre 500 et 5.10⁶ environ, et de préférence comprise entre 10³ et 3.10⁶ environ .

Parmi les polymères cationiques, on peut citer les protéines (ou hydrolysats de protéines) quaternisées et les polymères du type polyamine, polyaminoamide et polyammonium quaternaire. Ce sont des produits connus.

Les protéines ou hydrolysats de protéines quaternisés sont en particulier des polypeptides modifiés chimiquement portant en bout de chaîne, ou greffés sur celle-ci, des groupements ammonium quaternaire. Leur masse moléculaire peut varier par exemple de 1 500 à 10 000, et en particulier de 2 000 à 5 000 environ. Parmi ces composés, on peut citer notamment :
- les hydrolysats de collagène portant des groupements triéthylammonium tels que les produits dénommés dans le dictionnaire CTFA "TRIETHONIUM HYDROLYZED COLLAGEN ETHOSULFATE" ;
- les hydrolysats de collagène portant des groupements chlorure de triméthylammonium et de triméthylstéarylammonium, dénommés dans le dictionnaire CTFA "STEARTRIMONIUM HYDROLYZED COLLAGEN" ;
- les hydrolysats de protéines portant sur la chaîne polypeptidique des groupements ammonium quaternaire comportant au moins un radical alkyle ayant de 1 à 18 atomes de carbone.

Parmi ces hydrolysats de protéines, on peut citer entre autres le "CROQUAT L", le "CROQUAT M", le "CROQUAT S" et le "CROTEIN Q" vendus par la Société CRODA.

D'autre protéines ou hydrolysats quaternisés sont par exemple ceux vendus par la Société INOLEX, sous la dénomination "LEXEIN QX 3000".

On peut encore citer les protéines végétales quaternisées telles que les protéines de blé, de maïs ou de soja: comme protéines de blé quaternisées, on peut citer celles appelées dans le dictionnaire CTFA "COCODIMONIUM HYDROLYSED WHEAT PROTEIN", "LAURIDIMONIUM HYDROLYSED WHEAT PROTEIN", ou encore "STEARDIMONIUM HYDROLYSED WHEAT PROTEIN".

Les polymères du type polyamine, polyamidoamide, polyammonium quaternaire, utilisables conformément à la présente invention, pouvant être notamment mentionnés, sont ceux décrits dans les brevets français n°2 505 348 ou 2 542 997. Parmi ces polymères, on peut citer :
(1) les homopolymères ou copolymères dérivés d'esters ou d'amides acryliques ou méthacryliques et comportant au moins un des motifs de formules suivantes: 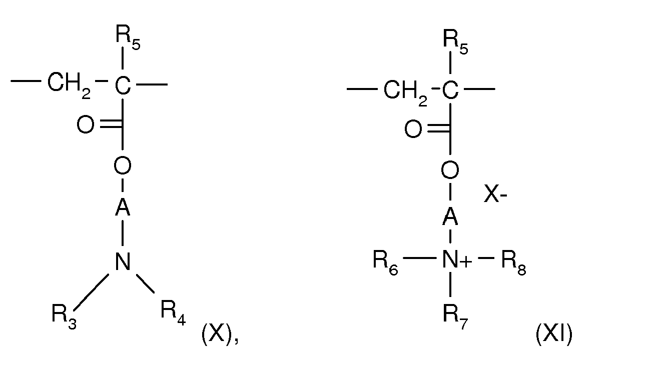 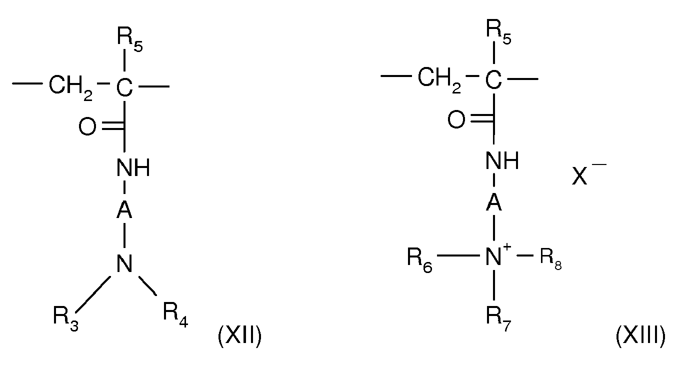 dans lesquelles:
   R₃ et R₄ , identiques ou différents, représentent hydrogène ou un groupe alkyle ayant de 1 à 6 atomes de carbone et de préférence méthyle ou éthyle;
   R₅ , identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₆, R₇, R₈, identiques ou différents, représentent un groupe alkyle ayant de 1 à 18 atomes de carbone ou un radical benzyle et de préférence un groupe alkyle ayant de 1 à 6 atomes de carbone;
   X désigne un anion dérivé d'un acide minéral ou organique tel que un anion méthosulfate ou un halogénure tel que chlorure ou bromure.
   Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :
   - les copolymères d'acrylamide et de diméthylaminoéthyl méthacrylate quaternisé au sulfate de diméthyle ou avec un hologénure de diméthyle tels que celui vendu sous la dénomination HERCOFLOC par la société HERCULES,
   - les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/vinylcaprolactame/ vinylpyrrolidone tel que le produit vendu sous la dénomination GAFFIX VC 713 par la société ISP,
   - les copolymère vinylpyrrolidone / méthacrylamidopropyl diméthylamine commercialisés notamment sous la dénomination STYLEZE CC 10 par ISP.
   - et les copolymères vinylpyrrolidone / méthacrylamide de diméthylamino-propyle quaternisé tel que le produit vendu sous la dénomination "GAFQUAT HS 100" par la société ISP.
   - Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE^{®} SC 92 » par la Société CIBA. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de « SALCARE^{®} SC 95 » et « SALCARE^{®} SC 96 » par la Société CIBA.
(2) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(3) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;
(4) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(5) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(6) les cyclopolymères d'alkyl diallyl amine ou de dialkyl diallyl ammonium tels que les homopolymères ou copolymères comportant comme constituant principal de la chaîne des motifs répondant aux formules (XIV) ou (XV) : 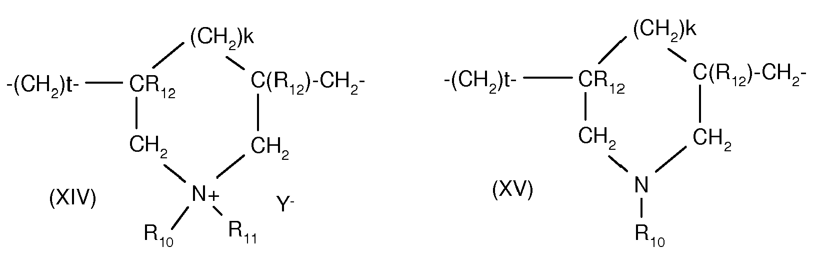 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄) ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   R₁₀ et R₁₁, indépendamment l'un de l'autre, désignent de préférence un groupement alkyle ayant de 1 à 4 atomes de carbone.
   Parmi les polymères définis ci-dessus, on peut citer plus particulièrement l'homopolymère de chlorure de diméthyldiallylammonium vendu sous la dénomination "Merquat 100" par la société NALCO (et ses homologues de faibles masses moléculaires moyenne en poids) et les copolymères de chlorure de diallyldiméthylammonium et d'acrylamide commercialisés sous la dénomination "MERQUAT 550".
(7) le polymère de diammonium quaternaire contenant des motifs récurrents répondant à la formule : 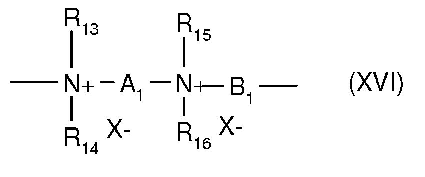 formule (XVI) dans laquelle :
   R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R_{13,} R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C₁-C₆ linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
   A₁ et B₁ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A₁, R₁₃ et R₁₅ peuvent former avec les deux atomes d'azote auxquels ils sont rattachés un cycle pipérazinique ; en outre si A₁ désigne un radical alkylène ou hydroxyalkylène linéaire ou ramifié, saturé ou insaturé, B₁ peut également désigner un groupement (CH₂)ₙₚ-CO-D-OC-(CH₂)p-
      p est un nombre entier variant de 2 à 20 environ
      dans lequel D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)ₓ -CH₂-CH₂-

         -[CH₂-CH(CH₃)-O]y-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH- ;
   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule : 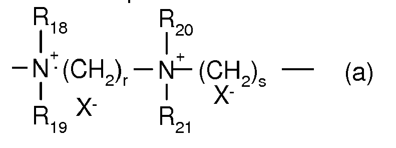 dans laquelle R₁₈, R₁₉, R₂₀ et R₂₁, identiques ou différents, désignent un radical alkyle ou hydroxyalkyle ayant de 1 à 4 atomes de carbone environ, r et s sont des nombres entiers variant de 2 à 20 environ et, X- est un anion dérivé d'un acide minéral ou organique.
   Un composé de formule (a) particulièrement préféré est celui pour lequel R₁₈, R₁₉, R₂₀ et R21, représentent un radical méthyle et r = 3, s = 6 et X = Cl, dénommé Hexadimethrine chloride selon la nomenclature INCI (CTFA).
(8) les polymères de polyammonium quaternaires constitués de motifs de formule (XVII): 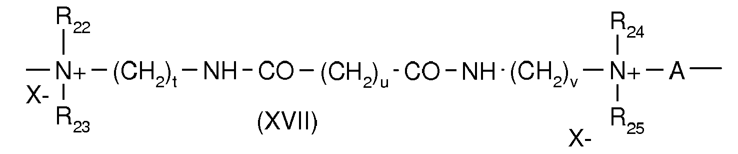 formule dans laquelle :
   R₂₂, R₂₃, R₂₄ et R₂₅, identiques ou différents, représentent un atome d'hydrogène ou un radical méthyle, éthyle, propyle, β-hydroxyéthyte, β-hydroxypropyle ou -CH₂CH₂(OCH₂CH₂)ₚOH,
   où p est égal à 0 ou à un nombre entier compris entre 1 et 6, sous réserve que R₂₂, R₂₃, R₂₄ et R₂₅ ne représentent pas simultanément un atome d'hydrogène,
   t et u, identiques ou différents, sont des nombres entiers compris entre 1 et 6,
   v est égal à 0 ou à un nombre entier compris entre 1 et 34,
   X⁻ désigne un anion tel qu'un halogènure,
   A désigne un radical d'un dihalogénure ou représente de préférence -CH₂-CH₂-O-CH₂-CH₂-.
   De tels composés sont notamment décrits dans la demande de brevet EP-A-122 324.
   On peut par exemple citer parmi ceux-ci, les produits "Mirapol® A 15", "Mirapol® AD1 ", "Mirapol® AZ1" et "Mirapol® 175" vendus par la société Miranol.
(9) Les polymères quaternaires de vinylpyrrolidone et de vinylimidazole tels que par exemple les produits commercialisés sous les dénominations Luviquat® FC 905, FC 550 et FC 370 par la société B.A.S.F.
(10) Les polysaccharides cationiques notamment les celluloses et les gommes de galactomannanes cationiques. Parmi les polysaccharides cationiques, on peut citer plus particulièrement les dérivés d'éthers de cellulose comportant des groupements ammonium quaternaires, les copolymères de cellulose cationiques ou les dérivés de cellulose greffés avec un monomère hydrosoluble d'ammonium quaternaire et les gommes de galactomannanes cationiques.

Les dérivés d'éthers de cellulose comportant des groupements amonium quaternaires décrits dans le brevet français 1 492 597. Ces polymères sont également définis dans le dictionnaire CTFA comme des ammonium quaternaires d'hydroxyéthylcellulose ayant réagi avec un époxyde substitué par un groupement triméthylammonium.

Les copolymères de cellulose cationiques ou les dérivés de cellulose greffés avec un monomère hydrosoluble d'ammonium quaternaire, sont décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.

Les gommes de galactomannane cationiques sont décrites plus particulièrement dans les brevets US 3 589 578 et 4 031 307 en particulier les gommes de guar contenant des groupements cationiques trialkylammonium. On utilise par exemple des gommes de guar modifiées par un sel (par ex. chlorure) de 2,3-époxypropyl triméthylammonium.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les dérivés d'éther de cellulose quaternaires tels que les produits commercialisés sous la dénomination « JR 400 » par la société UNION CARBIDE CORPORATION, les cyclopolymères, en particulier les homopolymères de sel de diallyldiméthylammonium et les copolymères de sel de diallyldiméthylammonium et d'acrylamide en particulier les chlorures, commercialisés sous les dénominations « MERQUAT 550 » et « MERQUAT S » par la société MERCK, les polysaccharides cationiques et plus particulièrement les gommes de guar modifiées par du chlorure de 2,3-époxypropyl triméthylammonium commercialisées par exemple sous la dénomination «JAGUAR C13S » par la société MEYHALL, les homopolymères et les copolymères éventuellement réticulés de sel de (méth)acryloyloxyéthyltriméthylammonium, vendus par la société ALLIED COLLOIDS en solution à 50% dans de l'huile minérale sous les dénominations commerciales SALCARE SC92 (copolymère réticulé du chlorure de méthacryloyloxyéthyltriméthylammonium et de l'acrylamide) et SALCARE SC95 (homopolymère réticulé du chlorure de méthacryloyloxyéthyl triméthylammonium), les copolymères quaternaires de vinylpyrrolidone et de sel de vinyl imidazole tels que les produits commercialisés par BASF sous les dénominations LUVIQUAT FC 370, LUVIQUAT FC 550, LUVIQUAT FC 905 et LUVIQUAT HM-552.

Les polymères cationiques additionnels sont utilisés dans les compositions de l'invention dans des proportions comprises entre 0,001 et 20% en poids et de préférence entre 0,05 et 5% en poids et plus particulièrement de 0,1 à 3% en poids par rapport au poids total de la composition.

Dans un mode de réalisation préféré de l'invention, les compositions selon la présente invention comprennent en outre une ou plusieurs silicones et notamment des polyorganosiloxanes modifiés ou non, à savoir des huiles de polyorganosiloxanes ou des gommes ou des résines de polyorganosiloxanes, telles quelles ou sous forme de solutions dans des solvants organiques ou encore sous forme d'émulsions ou de microémulsions.

Parmi les polyorganosiloxanes pouvant être utilisés conformément à la présente invention, on peut citer à titre non limitatif :
I. Les silicones volatiles : celles-ci possèdent un point d'ébullition compris entre 60°C et 260°C. Elles sont choisies parmi les silicones cycliques contenant de 3 à 7 atomes de silicium et de préférence 4 à 5. Il s'agit, par exemple, de l'octaméthyl-cyclotétrasiloxane vendu sous le nom de "VOLATILE SILICONE 7207" par UNION CARBIDE ou "SILBIONE 70045 V2" par RHONE POULENC, le décaméthylcyclopentasiloxane vendu sous le nom de "VOLATILE SILICONE 7158" par UNION CARBIDE, "SILBIONE 70045 V5 par RHONE POULENC, ainsi que leurs mélanges. On cite également les cyclocopolymères du type diméthylsiloxane/méthylalkylsiloxane, tel que la "SILICONE VOLATILE FZ 3109" vendue par la Société UNION CARBIDE, qui est un cyclocopolymère diméthylsiloxane/méthyloctylsiloxane.
II. Les silicones non volatiles : elles sont constituées principalement par:
   (i) les polyalkylsiloxanes ; Parmi les polyalkylsiloxanes, on peut citer principalement les polydiméthylsiloxanes linéaires à groupements terminaux triméthylsilyle, comme par exemple, et à titre non limitatif, les huiles "SILBIONE" de la série 70047 commercialisées par RHODIA CHIMIE; les huiles DC 200 de DOW CORNING, les PDMS à groupements terminaux hydroxydiméthylsilyle ;
   (ii) les polyarylsiloxanes ;
   (iii) les polyalkylarylsiloxanes ; on peut citer les polyméthylphénylsiloxanes, les polydiméthylméthylphénylsiloxanes, les polydiméthyldiphénylsiloxanes linéaires et ramifiés, tels que par exemple l'huile "RHODORSIL 70763" de RHODIA CHIMIE;
   (iv) les gommes de silicone ; ce sont des polydiorganosiloxanes de masse moléculaire comprise entre 200.000 et 5.000.000, utilisés seuls ou en mélange dans un solvant choisi parmi les silicones volatiles, les huiles polydiméthylsiloxanes (PDMS), les huiles polyphénylméthylsiloxanes, (PPMS), les isoparaffines, le chlorure de méthylène, le pentane, le dodécane, le tridécane, le tétradécane ou leurs mélanges; on cite, par exemple, les composés suivants :
      - gommes de polydiméthylsiloxane,
      - gommes de poly[(diméthylsiloxane)/(méthylvinylsiloxane)],
      - gommes de poly[(diméthylsiloxane)/(diphénylsiloxane)],
      - gommes de poly[(diméthylsiloxane))(phénylméthylsiloxane)],
      - gommes de poly[(diméthylsiloxane)/ (diphénylsiloxane)/(méthylvinylsiloxane)];

On peut aussi citer, par exemple, à titre non limitatif, les mélanges suivants :
1) les mélanges formés à partir d'une gomme polydiméthylsiloxane hydroxylé en bout de chaîne (DIMETHICONOL selon la nomenclature CTFA), et d'un polydiméthylsiloxane cyclique (CYCLOMETHICONE selon la nomenclature CTFA), tels que le produit "Q2 1401" vendu par la Société DOW CORNING;
2) les mélanges formés à partir d'une gomme polydiméthylsiloxane avec une silicone cyclique, tel que le produit "SF 1214 SILICONE FLUID" de GENERAL ELECTRIC, qui est une gomme SE 30 de PM 500 000 solubilisée dans la "SF 1202 SILICONE FLUID" (décaméthylcyclopentasiloxane);
3) les mélanges de deux PDMS de viscosité différente, notamment d'une gomme PDMS et d'une huile PDMS, tels que les produits "SF 1236" et "CF 1241" de la Société GENERAL ELECTRIC;
   (v) les résines de silicone ; de préférence des systèmes siloxaniques réticulés renfermant les unités R₂SiO_{2/2}, RSiO_{3/2} et SiO_{4/2} dans lesquelles R représente un groupement hydrocarboné possédant 1 à 6 atomes de carbone ou un groupement phényle. Parmi ces résines, on peut citer le produit vendu sous la dénomination "DOW CORNING 593";
   (vi) les polyorganosiloxanes organomodifiés ; c'est-à-dire des silicones telles que définies précédemment, comportant dans leur structure générale, un ou plusieurs groupements organofonctionnels directement fixés sur la chaîne siloxanique ou fixés par l'intermédiaire d'un radical hydrocarboné; on cite, par exemple, les silicones comportant :
      a) des groupements polyéthylèneoxy et/ou polypropylèneoxy, comportant éventuellement des groupes alkyle, tels que le produit dénommé diméthicone copolyol vendu par la Société DOW CORNING sous la dénomination "DC 1248", et l'alkyl (C12) méthicone copolyol vendu par la Société DOW CORNING sous la dénomination "Q2 5200";
      b) des groupements (per)fluorés comme les groupements trifluoroalkyle, telles que, par exemple, celles vendues par la Société GENERAL ELECTRIC sous les dénominations "FF.150 Fluorosilicone Fluid";
      c) des groupements hydroxyacylamino, telles que celles décrites dans la demande de brevet européen EP-A-0 342 834 et en particulier la silicone vendue par la Société DOW CORNING sous la dénomination "Q2-8413" ;
      d) des groupements thiols comme dans les silicones "X 2-8360" de DOW CORNING ou les "GP 72A" et "GP 71" de GENESEE ;
      e) des groupements aminés substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ou aminoalkyl(C₁-C₄)aminoalkyl(C₁-C₄). On utilise plus particulièrement les silicones dénommées amodiméthicone et triméthylsilylamodiméthicone selon la dénomination CTFA (1997) ;
      f) des groupements carboxylates, comme les produits décrits dans le brevet européen EP 186 507 de CHISSO CORPORATION;
      g) des groupements hydroxylés, comme les polyorganosiloxanes à fonction hydroxyalkyle, décrits dans la demande de brevet FR-A-2 589 476 ;
      h) des groupements alcoxylés comportant au moins 12 atomes de carbone comme le produit "SILICONE COPOLYMER F 755" de SWS SILICONES;
      i) des groupements acyloxyalkyle comportant au moins 12 atomes de carbone, comme par exemple les polyorganosiloxanes décrits dans la demande de brevet FR-A-2 641 185;
      j) des groupements ammonium quaternaire, comme dans le produit "ABIL K 3270" de la Société GOLDSCHMIDT ;
      k) des groupements amphotères ou bétaïniques, tels que dans le produit vendu par la Société GOLDSCHMIDT sous la dénomination "ABIL B 9950" ;
      l) des groupements bisulfite, tels que dans les produits vendus par la Société GOLDSCHMIDT sous les dénominations "ABIL S 201" et "ABIL S 255";
   (vii) les copolymères blocs ayant un bloc linéaire polysiloxane-polyalkylène comme unité répétitive ; la préparation de tels copolymères blocs mis en oeuvre dans le cadre de la présente invention est décrite dans la demande européenne EP 0 492 657 A1, dont l'enseignement est inclus à titre de référence dans la présente description;
   (viii) les polymères siliconés greffés, à squelette organique non siliconé, constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromonomère polysiloxane ; notamment ceux choisis plus préférentiellement parmi ceux décrits dans les brevets US 4.963.935, US 4.728.571 et US 4.972.037 et les demandes de brevet EP-A 0 412 704, EP-A-0 412 707, EP-A-0 640 105 et WO 95/00578 dont les enseignement sont totalement inclus dans la présente description à titre de références non limitatives;
   (ix) les polymères siliconés greffés, à squelette polysiloxanique greffé par des monomères organiques non siliconés, comprenant une chaîne principale de polysiloxane sur laquelle se trouve greffé, à l'intérieur de ladite chaîne ainsi qu'éventuellement à l'une au moins de ses extrémités, au moins un macromonomère organique ne comportant pas de silicone ; des exemples de tels polymères, ainsi que leur mode particulier de préparation, sont notamment décrits dans les demandes de brevets EP-A-0 582 152, WO 93/23009 et WO 95/03776 dont les enseignements sont inclus totalement dans la présente description à titre de références non limitatives;
   (x) ou leurs mélanges.

Les silicones préférées pour être utilisées selon l'invention sont les polyorganopolysiloxanes non volatils et préférentiellement les huiles ou gommes polydiméthylsiloxanes éventuellement aminés, arylés ou alkylarylés.

Les silicones sont utilisées dans les compositions de l'invention dans des proportions comprises entre 0,01 et 20% en poids et de préférence entre 0,1 et 10% en poids par rapport au poids total de la composition.

Selon la présente invention, les composés de type céramide sont notamment les céramides et/ou les glycocéramides et/ou les pseudocéramides et/ou les néocéramides, naturels ou synthétiques.

Des composés de type céramide sont par exemple décrits dans les demandes de brevet DE4424530, DE4424533, DE4402929, DE4420736, WO95/23807, WO94/07844, EP-A-0646572, WO95/16665, FR-2 673 179, EP-A-0227994 et WO 94/07844, WO94/24097, WO94/10131 dont les enseignements sont ici inclus à titre de référence.

Des composés de type céramides particulièrement préférés selon l'invention sont par exemple :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
- le (bis-(N-hydroxyéthyl N-cétyl) malonamide),
- le N-(2-hydroxyéthyl)-N-(3-cétyloxy-2-hydroxypropyl)amide d'acide cétylique
- le N-docosanoyl N-méthyl-D-glucamine
ou les mélanges de ces composés.

Les esters d'acide carboxylique insolubles dans l'eau selon l'invention sont insolubles dans l'eau à une concentration supérieure ou égale à 0,1% en poids dans l'eau à 25°C, c'est à dire qu'ils ne forment pas dans ces conditions une solution isotrope transparente à l'oeil nu.

Les esters utilisables dans la présente invention sont de préférence monomériques. Ils sont de préférence non ioniques, non siliconés .

Les esters d'acide carboxylique insolubles dans l'eau selon l'invention peuvent contenir des groupements hydroxyles.

Le nombre total de carbone des esters de l'invention va de préférence de 12 à 50 et plus particulièrement de 16 à 40 et encore mieux de 16 à 30.

Selon l'invention, les esters utilisés sont de préférence liquides à température ambiante (25°C) et à pression atmosphérique (1 atm).

Les esters carboxyliques sont de préférence choisis parmi :
1) les esters d'acide carboxylique en C3-C30 aliphatique ou alicyclique et d'alcool en C1-C30, l'un au moins de l'acide ou de l'alcool étant ramifié ou possédant au moins une double-liaison carbone-carbone, et
2) les esters d'acide aromatique en C7-C30 dont la fonction carboxylique est directement liée au cycle aromatique et d'alcool en C1-C30,

Les esters d'acide carboxylique en C3-C30 et d'alcool en C1-C30, l'un au moins de l'acide ou de l'alcool étant ramifié ou insaturé, sont notamment choisis parmi les esters d'acide carboxylique en C6-C24 et d'alcool en C3-C20.

Les esters selon l'invention sont notamment choisis parmi :
les esters d'acide carboxylique linéaire ayant de 12 à 26 atomes de carbone et d'alcool ramifié ayant de 3 à 12 atomes de carbone,
les esters d'acide carboxylique linéaire ayant de 2 à 12 atomes de carbone et d'alcool ramifié ayant de 8 à 26 atomes de carbone.
les esters d'acide carboxylique ramifié ayant de 8 à 26 atomes de carbone, de préférence 8 à 12 et d'alcool ramifié ayant de 8 à 26 atomes de carbone, de préférence 8 à 12.

On peut citer le béhénate d'octyldodécyle ; le béhénate d'isocétyle ; le lactate d'isocétyle ; le lactate d'isostéaryle ; l'octanoate de isostéaryle ; l'octanoate d'isocétyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; l'isostéarate de myristyle ; l'isononanoate d'octyle ; l'isononate de 2-éthylhexyle; l'isostéarate d'octyle ; l'érucate d'octyldodécyle ; le palmitate d'isopropyle, le palmitate d'éthyl-2-héxyle, le palmitate de 2-octyldécyle, les myristates d'alkyles ramifiés tels que le myristate d'isopropyle, de t-butyle, de 2-octyldodécyle, l'isostéarate d'hexyle, l'isostéarate de butyle, le stéarate d'isobutyle ; le laurate de 2-hexyldécyle.

De préférence, l'acide et l'alcool de l'ester sont saturés.

On peut également utiliser les esters liquides d'acide carboxylique ramifié ayant de 4 à 6 atomes de carbone et d'alcool ayant de 8 à 26 atomes de carbone.

Ces esters liquides ramifiés selon l'invention ont de préférence la formule suivante :

R₁ COOR₂ (VI)

dans laquelle :
R₁ désigne un radical hydrocarboné, ramifié, éventuellement mono ou polyhydroxylé, ayant de 3 à 5 atomes de carbone,
R₂ désigne un radical hydrocarboné, linéaire ou ramifié, éventuellement mono ou polyhydroxylé, ayant de 12 à 26 atomes de carbone, de préférence ayant de 16 à 22 atomes de carbone.

R₁ désigne de préférence un radical alkyle ramifié ayant de 3 à 5 atomes de carbone, et plus particulièrement un radical tertio-butyle.

R₂ désigne de préférence un radical alkyle saturé ou insaturé ayant 12 à 26 atomes de carbone, plus particulièrement ramifié et encore plus particulièrement choisi parmi les radicaux tridécyle, isocétyle, isostéaryle, octyldodécyle et isoarachidyle.

Les esters liquides ramifiés particulièrement préférés sont le néopentanoate d'isostéaryle (formule (VI) dans laquelle R₁=tertio-butyle et R₂=isostéaryle), le néopentanoate de tridécyle, le néopentanoate d'isocétyle, et le néopentanoate d'isoarachidyle.

De préférence, l'acide et l'alcool de l'ester sont saturés. De préférence l'alcool est un monoalcool (une seule fonction hydroxyle).

Parmi les esters cités ci-dessus, on préfère utiliser le palmitate d'isopropyle, le palmitate d'éthyl-2-héxyle, le palmitate de 2-octyldécyle, les myristates d'alkyles ramifiés tels que le myristate d'isopropyle, de t-butyle, de 2-octyldodécyle, l'isostéarate d'hexyle, le isostéarate de butyle, le stéarate d'isobutyle ; le laurate de 2-hexyldécyle, le néopentanoate d'isostéaryle, néopentanoate de tridécyle et l'isononanate d'isononyle.

Les esters d'acide aromatique en C7-C30 et d'alcool en C1-C30 sont de préférence des esters d'acide aromatique en C7-C17 et d'alcool en C1-C20. Ces esters sont notamment les benzoates d'alkyle en C12-C15, le benzoate d'isostéaryle, le benzoate d'octyledodécyle, le benzoate de béhényle, le benzoate d'éthyl-2hexyle.

Selon l'invention, les agents conditionneurs peuvent représenter de 0,001 % à 10 % en poids, de préférence de 0,005% à 5% en poids, et encore plus préférentiellement de 0,01 % à 3 % en poids, du poids total de la composition finale.

Le milieu physiologiquement acceptable est de préférence constitué par de l'eau ou un mélange d'eau et de solvants cosmétiquement ou dermatologiquement acceptables tels que des monoalcools, des polyols, des éthers de polyols qui peuvent être utilisés seuls ou en mélange. L'eau représente de préférence de 30 à 98% en poids et de préférence de 50 à 98% en poids par rapport au poids total de la composition.

On peut citer plus particulièrement les monoalcools tels que l'éthanol, l'isopropanol, les polyols tels que le diéthylèneglycol, la glycérine et les éthers de polyols tels que les éthers de diéthylèneglycol.

La composition de l'invention peut également contenir au moins un additif choisi parmi parmi les séquestrants, les adoucissants, les modificateurs de mousse, les colorants, les agents nacrants, les agents hydratants, les agents antipelliculaires ou anti-séborrhéiques, les agents anti-chute ou repousse des cheveux, les agents de mise en suspension, les acides gras, les parfums, les conservateurs, les filtres solaires, les protéines et les hydrolysats de protéines non cationiques, les vitamines et provitamines, les polymères anioniques, non ioniques ou amphotères, les agents alcalinisants ou acidifiants, les humectants, les sucres, les polymères anioniques, non ionique ou amphotères, le menthol, les dérivés de nicotinates, les stabilisateurs de mousse, les agents propulseurs, les vitamines, et les provitamines et tout autre additif classiquement utilisé dans le domaine cosmétique.

Ces additifs sont présents dans la composition selon l'invention dans des proportions pouvant aller de 0 à 40% en poids par rapport au poids total de la composition. La quantité précise de chaque additif est fonction de sa nature et est déterminée facilement par l'homme de l'art.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions conformes à l'invention peuvent être plus particulièrement utilisées pour le lavage ou le traitement des matières kératiniques telles que les cheveux, la peau, les cils, les sourcils, les ongles, les lèvres, le cuir chevelu et plus particulièrement les cheveux.

Les compositions selon l'invention sont utilisées généralement comme produits notamment pour le lavage, le soin, le conditionnement, le maintien de la coiffure ou la mise en forme des matières kératiniques telles que les cheveux.

Les compositions de l'invention peuvent plus particulièrement se présenter sous forme de shampooing, d'après-shampooing à rincer ou non, de compositions pour permanente, défrisage, coloration ou décoloration, ou encore sous forme de compositions à appliquer avant ou après une coloration, une décoloration, une permanente ou un défrisage ou encore entre les deux étapes d'une permanente ou d'un défrisage.

Dans une première variante préférée, les compositions sont des compositions lavantes et moussantes pour les cheveux et/ou la peau.

En particulier, les compositions selon l'invention sont des compositions détergentes moussantes telles que des shampooings, des gels-douche et des bains moussants, des produits démaquillants. Dans ce mode de réalisation de l'invention, les compositions comprennent au moins un ou plusieurs tensioactifs détergents.

Le ou les tensioactifs peuvent alors être indifféremment choisis, seuls ou en mélanges, au sein des tensioactifs anioniques, amphotères, non ioniques notamment tels que définis ci-dessus.

Dans les compositions conformes à l'invention sous forme de compositions détergentes en particulier les shampooings, on utilise de préférence au moins un ou plusieurs tensioactifs anioniques ou des mélanges d'au moins un ou plusieurs tensioactifs anioniques et d'au moins un ou plusieurs tensioactifs amphotères ou d'au moins un ou plusieurs tensioactifs non ioniques.

Un mélange particulièrement préféré est un mélange comprenant au moins un agent tensioactif anionique et au moins un agent tensioactif amphotère.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélange avec :
- soit un agent tensioactif amphotère tel que les dérivés d'amine dénommés disodiumcocoamphodipropionate ou sodiumcocoamphopropionate commercialisés notamment par la société RHODIA CHIMIE sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylbétaïnes ou les alkylamidobétaïnes et en particulier la cocobétaïne commercialisée sous la dénomination "DEHYTON AB 30" en solution aqueuse à 32 % de MA par la société COGNIS.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélange avec :
- soit un agent tensioactif amphotère tel que les dérivés d'amine dénommés disodiumcocoamphodipropionate ou sodiumcocoamphopropionate commercialisés notamment par la société RHODIA CHIMIE sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylbétaïnes en particulier la cocobétaïne commercialisée sous la dénomination "DEHYTON AB 30" en solution aqueuse à 32 % de MA par la société COGNIS et la cocoamidopropylbétaïne vendue notamment par GOLDSCHMIDT sous le nom de TEGOBETAINE F50.

La quantité minimale en tensioactif est alors celle juste suffisante pour conférer à la composition finale un pouvoir moussant et/ou détergent satisfaisant.

Ainsi, selon l'invention, le tensioactif détergent peut représenter de 3 % à 30 % en poids, de préférence de 6 % à 25 % en poids, et encore plus préférentiellement de 8 % à 20 % en poids, du poids total de la composition finale.

Le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée.

Les modifications de la méthode sont les suivantes :
La mesure se fait à la température de 22°C avec de l'eau osmosée. La concentration de la solution est de 2g/l. La hauteur de la chute est de 1 m. La quantité de composition qui chute est de 200 ml. Ces 200 ml de composition tombe dans une éprouvette ayant un diamètre de 50 mm et contenant 50 ml de la composition à tester. La mesure est faite 5 minutes après l'arrêt de l'écoulement de la composition.

Dans une deuxième variante préférée, lorsque la composition se présente sous la forme d'un après-shampooing éventuellement à rincer, elle comprend avantageusement un ou plusieurs tensioactifs cationiques, la concentration des dits tensioactifs allant généralement de 0,1 à 10% en poids et de préférence de 0,5 à 5% en poids par rapport au poids total de la composition.

Les compositions de l'invention peuvent également se présenter sous forme de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Les compositions selon l'invention peuvent se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau après un éventuel temps de pause.

L'invention va être maintenant plus complètement illustrée à l'aide des exemples suivants qui ne sauraient être considérés comme la limitant aux modes de réalisation décrits. Dans ce qui suit, MA signifie Matière Active.

Les exemples qui suivent sont destinés à illustrer l'invention.

### EXEMPLE I :

On a préparé la composition de shampooing suivante :

| | |
|---|---|
| Lauryl éther sulfate de sodium (2.2 OE) en solution aqueuse à 30% MA (TEXAPON AOS 225 UP de COGNIS) | 14,9 gMA |
| Cocoamidoéthyl (N-hydroxyéthyl, N-carboxyméthyl) glycinate de sodium en solution aqueuse à 30% MA (MIRANOL C2M CONC. NP deRHODIA) | 2,4 gMA |
| Copolymère d'esters d'acide acrylique ou méthacrylique, de méthacrylate de C1-4 dialkylamino C1-6 alkyl, de PEG/PPG-30/5 allyl ether, de méthacrylate d'alkyl éther de PEG 20-25 C10-30, de méthacrylate d'hydroxy C2-6 alkyl réticulé avec de l'éthylène glycol diméthacrylate en émulsion à 20% dans l'eau (CARBOPOL AQUA CC POLYMER de NOVEON) | 1 gMA |
| Carboxyméthylamidon de pomme de terre, sel de sodium faiblement réticulé (PRIMOJEL DE AVEBE) | 5,65 gMA |
| Acide lactique | 0,38 g |
| Sel di-sodique de ponceau SX (CI: 14 700) | 0,00015 g |
| Acide salicylique en poudre | 0,2 g |
| P-hydroxybenzoate d'ethyle | 0,15 g |
| Benzoate de sodium | 0,5 g |
| P-hydroxybenzoate de methyle, sel de sodium | 0,4 g |
| Parfum | 0,5 g |
| Hydroxyde de sodium pur ou Acide citrique, 1 H2O | Qs pH 5,3 |
| Eau desionisée | Qsp 100 g |
| | |

Cette composition présente une texture épaissie stable dans le temps. Elle s'applique et se répartit aisément sur les cheveux avec un bon démarrage de mousse. Cette composition s'élimine facilement au rinçage et confèrent aux cheveux traités douceur, lissage et brillance.

### EXEMPLE 2 :

On a préparé la composition de shampooing suivante :

| | |
|---|---|
| Lauryl éther sulfate de sodium (2.2 OE) en solution aqueuse à 30% MA (TEXAPON AOS 225 UP de COGN IS) | 14,9 gMA |
| Cocoamidoéthyl (N-hydroxyéthyl, N-carboxyméthyl) glycinate de sodium en solution aqueuse à 30% MA (MIRANOL C2M CONC. NP deRHODIA) | 2,4 gMA |
| Copolymère d'esters d'acide acrylique ou méthacrylique, de méthacrylate de C1-4 dialkylamino C1-6 alkyl, de PEG/PPG-30/5 allyl ether, de méthacrylate d'alkyl éther de PEG 20-25 C10-30, de méthacrylate d'hydroxy C2-6 alkyl réticulé avec de l'éthylène glycol diméthacrylate en émulsion à 20% dans l'eau (CARBOPOL AQUA CC de NOVEON) | 1 gMA |
| Carboxyméthylamidon de pomme de terre, sel de sodium faiblement réticulé (PRIMOJEL DE AVEBE) | 5,65 gMA |
| Hydroxyéthylcellulose quaternisée (JR 400 de AMERCHOL) | 0,5 g |
| Acide lactique | 0,38 |
| Sel di-sodique de ponceau SX (CI: 14 700) | 0,00015 |
| Acide salicylique en poudre | 0,2 |
| P-hydroxybenzoate d'ethyle | 0,15 |
| Benzoate de sodium | 0,5 |
| P-hydroxybenzoate de methyle, sel de sodium | 0,4 |
| Parfum | 0,5 |
| Hydroxyde de sodium pur ou Acide citrique, 1 H2O | Qs pH 5,3 |
| Eau desionisée | Qsp 100 g |
| | |

Cette composition présente une texture épaissie stable dans le temps. Elle s'applique et se répartit aisément sur les cheveux avec un bon démarrage de mousse. Cette composition s'élimine facilement au rinçage et confèrent aux cheveux traités douceur, lissage et brillance.

### EXEMPLE 3 :

On a préparé la composition de shampooing suivante :

| | |
|---|---|
| Lauryl éther sulfate de sodium (2.2 OE) en solution aqueuse à 30% MA (TEXAPON AOS 225 U P de COGN IS) | 12,83 gMA |
| Cocoamidoéthyl (N-hydroxyéthyl, N-carboxyméthyl) glycinate de sodium en solution aqueuse à 30% MA (MIRANOL C2M CONC. NP deRHODIA) | 0,6 gMA |
| Cocoyl bétaine en solution aqueuse à 30% MA (DEHYTON AB 30 de) | 1,57 gMA |
| Copolymère d'esters d'acide acrylique ou méthacrylique, de méthacrylate de C1-4 dialkylamino C1-6 alkyl, de PEG/PPG-30/5 allyl ether, de méthacrylate d'alkyl éther de PEG 20-25 C10-30, de méthacrylate d'hydroxy C2-6 alkyl réticulé avec de l'éthylène glycol diméthacrylate en émulsion à 20% dans l'eau (CARBOPOL AQUA CC de NOVEON) | 0,6 gMA |
| Phosphate de diamidon de mais (STRUCTURE ZEA de NATIONAL STARCH) | 3 g |
| Distéarate d'éthylène glycol | 2 g |
| Propylèneglycol | 2 g |
| Acide lactique | 0,38 |
| Sel di-sodique de ponceau SX (CI: 14 700) | 0,00015 |
| Acide salicylique en poudre | 0,2 |
| P-hydroxybenzoate d'ethyle | 0,15 |
| Benzoate de sodium | 0,5 |
| P-hydroxybenzoate de methyle, sel de sodium | 0,4 |
| Parfum | 0,5 |
| Hydroxyde de sodium pur ou Acide citrique, 1 H2O | Qs pH 5,3 |
| Eau desionisée | Qsp 100 g |

Cette composition présente une texture épaissie stable dans le temps. Elle s'applique et se répartit aisément sur les cheveux avec un bon démarrage de mousse. Cette composition s'élimine facilement au rinçage et confèrent aux cheveux traités douceur, lissage et brillance.

### EXEMPLE 4 :

On a préparé la composition de shampooing suivante :

| | |
|---|---|
| Lauryl éther sulfate de sodium (2.2 OE) en solution aqueuse à 30% MA (TEXAPON AOS 225 U P de COGN IS) | 12,83 gMA |
| Cocoamidoéthyl (N-hydroxyéthyl, N-carboxyméthyl) glycinate de sodium en solution aqueuse à 30% MA (MIRANOL C2M CONC. NP deRHODIA) | 0,6 gMA |
| Cocoyl bétaine en solution aqueuse à 30% MA (DEHYTON AB 30 de) | 1,57 gMA |
| Copolymère d'esters d'acide acrylique ou méthacrylique, de méthacrylate de C1-4 dialkylamino C1-6 alkyl, de PEG/PPG-30/5 allyl ether, de méthacrylate d'alkyl éther de PEG 20-25 C10-30, de méthacrylate d'hydroxy C2-6 alkyl réticulé avec de l'éthylène glycol diméthacrylate en émulsion à 20% dans l'eau (CARBOPOL AQUA CC de NOVEON) | 0,6 gMA |
| Amidon de tapioca modifié, réticulé et prégélatinisé (ULTRA-SPERSE 3de NATIONAL STARCH) | 3 g |
| Huile d'olive vierge | 0,5 g |
| Distéarate d'éthylène glycol | 2 g |
| Acide lactique | 0,38 |
| Sel di-sodique de ponceau SX (CI: 14 700) | 0,00015 |
| Acide salicylique en poudre | 0,2 |
| P-hydroxybenzoate d'ethyle | 0,15 |
| Benzoate de sodium | 0,5 |
| P-hydroxybenzoate de methyle, sel de sodium | 0,4 |
| Parfum | 0,5 |
| Hydroxyde de sodium pur ou Acide citrique, 1 H2O | Qs pH 5,3 |
| Eau desionisée | Qsp 100 g |
| | |

Cette composition présente une texture épaissie stable dans le temps. Elle s'applique et se répartit aisément sur les cheveux avec un bon démarrage de mousse. Cette composition s'élimine facilement au rinçage et confèrent aux cheveux traités douceur, lissage et brillance.

### EXEMPLE 5 :

On a préparé la composition de shampooing suivante :

| | |
|---|---|
| Lauryl éther sulfate de sodium (2.2 OE) en solution aqueuse à 30% MA (TEXAPON AOS 225 U P de COGN IS) | 12,83 gMA |
| Cocoamidoéthyl (N-hydroxyéthyl, N-carboxyméthyl) glycinate de sodium en solution aqueuse à 30% MA (MIRANOL C2M CONC. NP deRHODIA) | 0,6 gMA |
| Cocoyl bétaine en solution aqueuse à 30% MA (DEHYTON AB 30 de) | 1,57 gMA |
| Copolymère d'esters d'acide acrylique ou méthacrylique, de méthacrylate de C1-4 dialkylamino C1-6 alkyl, de PEG/PPG-30/5 allyl ether, de méthacrylate d'alkyl éther de PEG 20-25 C10-30, de méthacrylate d'hydroxy C2-6 alkyl réticulé avec de | 0,6 gMA |
| l'éthylène glycol diméthacrylate en émulsion à 20% dans l'eau (CARBOPOL AQUA CC POLYMER de NOVEON) | |
| Amidon de tapioca modifié, réticulé et prégélatinisé (ULTRA-SPERSE 3 de NATIONAL STARCH) | 3 g |
| Chlorure d'hydroxypropyl guar triméthylammonium (JAGUAR EXCEL de RHODIA) | 0,2 g |
| Distéarate d'éthylène glycol | 2 g |
| Acide lactique | 0,38 |
| Sel di-sodique de ponceau SX (CI: 14 700) | 0,00015 |
| Acide salicylique en poudre | 0,2 |
| P-hydroxybenzoate d'ethyle | 0,15 |
| Benzoate de sodium | 0,5 |
| P-hydroxybenzoate de methyle, sel de sodium | 0,4 |
| Parfum | 0,5 |
| Hydroxyde de sodium pur ou Acide citrique, 1 H2O | Qs pH 5,3 |
| Eau desionisée | Qsp 100 g |
| | |

Cette composition présente une texture épaissie stable dans le temps. Elle s'applique et se répartit aisément sur les cheveux avec un bon démarrage de mousse. Cette composition s'élimine facilement au rinçage et confèrent aux cheveux traités douceur, lissage et brillance.

### EXEMPLE 6

On a préparé la composition suivantes d'après-shampooing à rincer :

| | |
|---|---|
| Alcool stéarylique | 2 g |
| Copolymère d'esters d'acide acrylique ou méthacrylique, de méthacrylate de C1-4 dialkylamino C1-6 alkyl, de PEG/PPG-30/5 allyl ether, de méthacrylate d'alkyl éther de PEG 20-25 C10-30, de méthacrylate d'hydroxy C2-6 alkyl réticulé avec de l'éthylène glycol diméthacrylate en émulsion à 20% dans l'eau (CARBOPOL AQUA CC de NOVEON) | 0,8 gMA |
| Chlorure de béhényl triméthyl ammonium en solution aqueuse à 80% de MA (GENAMIN KDMP de CLARIANT) | 1,6 gMA |
| QUATERNIUM-87 en solution à 75%MA dans le propylène glycol (VARISOFT W 575 PG deGOLDSCHMIDT) | 2,51 gMA |
| Polydiméthylsiloxane à groupements aminoéthyl iminopropyl en émulsion aqueuse non ionique à 20% de MA (SME 253 de MOMENTIVE PERFORMANCE MATERIALS) | 0,8 gMA |
| Phosphate de di-amidon de maïs hydroxypropylé et prégélatinisé (STRUCTURE ZEA de NATIONAL STARCH) | 2 g |
| Cire de candelilla (Candelilla wax SP75 de STRAHL & PITSCH)= | 0,4 g |
| Parfum, conservateurs | qs |
| Acide lactique qs | pH 4 |
| Eau qsp | 100 g |

Cette composition stable de texture agréable a été appliquées sur des cheveux très sensibilisés. Elle s'élimine aisément lors du rinçage. Les propriétés cosmétiques des cheveux traités (démêlage, lissage, souplesse) sont excellentes et homogènes des racines aux pointes des cheveux. Les pointes ne sont pas fourchues.

Entre deux applications, les cheveux restent doux, souples et lisses.

### EXEMPLE 7

On a préparé la composition suivante d'après-shampooing à rincer :

| | |
|---|---|
| Copolymère d'esters d'acide acrylique ou méthacrylique, de méthacrylate de C1-4 dialkylamino C1-6 alkyl, de PEG/PPG-30/5 allyl ether, de méthacrylate d'alkyl éther de PEG 20-25 C10-30, de méthacrylate d'hydroxy C2-6 alkyl réticulé avec de l'éthylène glycol diméthacrylate en émulsion à 20% dans l'eau (CARBOPOL AQUA CC de NOVEON) | 0,25 gMA |
| Chlorure de béhényl triméthyl ammonium en solution aqueuse à 80% de MA (GENAMIN KDMP de CLARIANT) | 1,6 gMA |
| Emulsion cationique à 67% MA de copolymère polydiméthylsiloxane à groupements alpha-oméga vinyle / polydiméthylsiloxane à groupements alpha-oméga hydrogéno (DC-1997 de DOW CORNING) | 0,8 gMA |
| 2-hydroxypropyléther guar (JAGUAR HP 105 de RHODIA) | 0,3 g |
| Phosphate de di-amidon de maïs hydroxypropylé et prégélatinisé (STRUCTURE ZEA de NATIONAL STARCH) | 7,52 gMA |
| Mica-oxyde de titane (67,5/32,5) | 0,4 g |
| Parfum, conservateurs | qs |
| Acide lactique qs | pH 4 |
| Eau qsp | 100 g |

Cette composition stable de texture agréable a été appliquée sur des cheveux très sensibilisés. Elle s'élimine aisément lors du rinçage. Les propriétés cosmétiques des cheveux traités (démêlage, lissage, souplesse) sont excellentes et homogènes des racines aux pointes des cheveux. Les pointes ne sont pas fourchues.

Entre deux applications, les cheveux restent doux, souples et lisses.

## Revendications

1. Composition cosmétique, comprenant dans un milieu cosmétiquement acceptable :
(i) un ou plusieurs polymères cationiques qui sont produits par polymérisation d'un mélange de monomères comprenant :
a) un ou plusieurs monomères vinyliques substitués par un ou plusieurs groupes amino,
b) un ou plusieurs monomères vinyliques non ioniques hydrophobes,
choisis parmi ceux de formules (I) et (II) :
(I) CH₂=C(X)Z,
(II) CH₂=CH-OC(O)R;
dans lesquelles formules (I) et (II) :
X représente H ou un groupe méthyle ;
Z est choisi parmi les groupes -C(O)OR¹, -C(O)NH₂, -C(O)NHR¹, - C(O)N(R¹)₂, -C₆H₅, -C₆H₄R¹, -C₆H₄OR¹, -C₆H₄Cl, -CN, -NHC(O)CH₃, -NHC(O)H, N-(2-pyrrolidonyle), N-caprolactamyle, -C(O)NHC(CH₃)₃, -C(O)NHCH₂CH₂-NH-CH₂CH₂-urée, -SiR₃, -C(O)O(CH₂)ₓSiR₃, -C(O)NH(CH₂)ₓSiR₃ et -(CH₂)ₓSiR₃ ;
x représente un nombre entier allant de 1 à 6 ;
chaque R représente indépendamment un groupe alkyle en C₁-C₃₀ ; chaque R¹ représente indépendamment un groupe alkyle en C₁-C₃₀ , un groupe alkyle en C₂-C₃₀ hydroxylé, ou un groupe alkyle en C₁-C₃₀ halogéné et
c) un ou plusieurs monomères vinyliques associatifs,
e) un ou plusieurs monomères vinyliques non ioniques hydroxylés, et,
(ii) un ou plusieurs amidons.

2. Composition selon la revendication 1, **caractérisé en ce que** le ou les monomères vinyliques substitués par un ou plusieurs groupes amino sont choisis parmi :
- les mono-(C₁-C₄)alkylamino(C₁-C₈)alkyl(méth)acrylates,
- les di-(C₁-C₄)alkylamino(C₁-C₈)alkyl(méth)acrylates, de préférence les di-(C₁-C₄)alkylamino(C₁-C₆)alkyl(méth)acrylates,
- les mono-(C₁-C₄)alkylamino(C₁-C₈)alkyl(méth)acrylamides,
- les di-(C₁-C₄)alkylamino(C₁-C₈)alkyl(méth)acrylamides,
- les (méth)acrylamides hétérocycliques comprenant un atome d'azote,
- les (méth)acrylates hétérocycliques comprenant un atome d'azote,
et leurs mélanges.

3. Composition selon la revendication 2 **caractérisé en ce que** le ou les monomères vinyliques substitués par un ou plusieurs groupes amino sont choisis parmi :
- les (méth)acrylates de mono- ou di-(alkyl en C₁-C₄)amino(alkyle en C₁-C₄), tels que le (méth)acrylate de 2-(N,N-diméthylamino)éthyle, le (méth)acrylate de 3-(N,N-diméthylamino)propyle, le (méth)acrylate de 4-(N,N-diméthylamino)butyle, le (méth)acrylate de (N,N-diméthylamino)-t-butyle, le (méth)acrylate de 2-(N,N-diéthylamino)éthyle, le (méth)acrylate de 3-(N,N-diéthylamino)propyle, le (méth)acrylate de 4-(N,N-diéthylamino)butyle, le (méth)acrylate de 2-(N,N-dipropylamino)éthyle, le (méth)acrylate de 3-(N,N-dipropylamino)propyle et le (méth)acrylate de 4-(N,N-dipropylamino)butyle ;
- les mono- ou di-(alkyl en C₁-C₄)amino(alkyl en C₁-C₄)-(méth)acrylamides tels que le N'-(2-N,N-diméthylamino)éthyl (méth)acrylamide et le N'-(3-N,N-diméthylamino)propyl acrylamide ;
- les (méth)acrylamides ou (méth)acrylates à groupement hétérocyclique comprenant un atome d'azote, tels que le N-(2-pyridyl)acrylamide, le N-(2-imidazolyl)méthacrylamide, le méthacrylate de 2-(4-morpholinyl)éthyle, le acrylate de 2-(4-morpholinyl)éthyle, le N-(4-morpholinyl)méthacrylamide et le N-(4-morpholinyl)acrylamide ; et
- les hétérocycles azotés à groupement(s) vinyle(s) tels que la 2-vinylpyridine et la 4-vinylpyridine.

4. Composition selon l'une quelconque des revendications 1 à 3, **caractérisée en ce que** le ou les monomères vinyliques substitués par un ou plusieurs groupes amino représentent généralement de 10 à 70 % en poids, de préférence de 20 à 60 % en poids, par rapport au poids total du mélange de monomères.

5. Composition selon l'une quelconque des revendications précédentes, **caractérisée en ce que** le ou les monomères vinyliques non ioniques hydrophobes sont choisis parmi les (méth)acrylates d'alkyle en C₁-C₃₀, les (alkyl en C₁-C₃₀)(méth)acrylamides, le styrène, les styrènes substitués, les esters de vinyle, les nitriles insaturés, et les silanes insaturés.

6. Composition selon l'une quelconque des revendications précédentes, **caractérisée en ce que** le ou les monomères vinyliques non ioniques hydrophobes représentent généralement de 20 à 80% en poids, de préférence de 20 à 70% en poids, par rapport au poids total du mélange de monomères.

7. Composition selon l'une quelconque des revendications précédentes, **caractérisée en ce que** le ou les monomères vinyliques associatifs sont choisis parmi les composés de formule (III) : 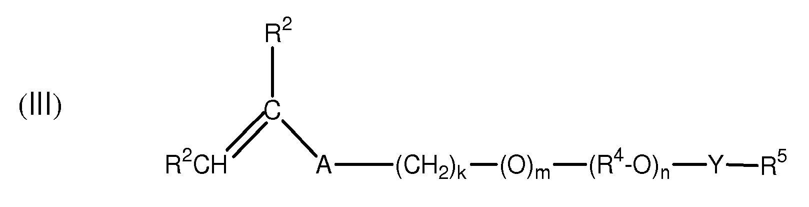 dans laquelle
chaque R² est indépendamment H, un groupe méthyle, un groupe -C(O)OH, ou un groupe -C(O)OR³ ;
R³ est un alkyle en C₁-C₃₀ ;
A est un groupe -CH₂C(O)O-, -C(O)O-, -O-, CH₂O, -NHC(O)NH-, - C(O)NH-, -Ar-(CE₂)_{z}-NHC(O)O-, -Ar-(CE₂)_{z} -NHC(O)NH-, ou -CH₂CH₂-NHC(O)- ;
Ar est un groupe aryle divalent ;
E est H ou un groupe méthyle ;
z est égal à 0 ou 1 ;
k est un entier variant de 0 à 30 ;
m est égal à 0 ou 1, à la condition que lorsque k = 0, m = 0, et lorsque k varie de 1 à 30, m est égal à 1 ;
(R⁴-O)ₙ est un polyoxyalkylène, qui est un homopolymère, un copolymère aléatoire, ou un copolymère à blocs, avec des unités oxyalkylène en C₂-C₄,
R⁴ est C₂H₄, C₃H₆, C₄H₈, ou leurs mélanges,
n est un entier variant de 5 à 250,
Y est -R⁴O-, -R⁴NH-, -C(O)-, -C(O)NH-, R⁴NHC(O)NH-, ou -C(O)NHC(O)-;
R⁵ est un alkyle substitué ou non choisi parmi les alkyles linéaires en C₈-C₄₀, les alkyles ramifiés en C₈-C₄₀, les alicycliques en C₈-C₄₀, les phényles substitués par un groupe alkyle en C₂-C₄₀, les alkyles en C₂-C₄₀ substitués par un groupe aryle, les esters complexes en C₈-C₈₀,
le groupe alkyle R⁵ comprenant éventuellement un ou plusieurs substituants choisis parmi les groupes hydroxyle, alcoxyle et halogèno.

8. Composition selon la revendication 7, **caractérisée en ce que** le ou les monomères vinyliques associatifs sont choisis parmi les (méth)acrylates polyéthoxylés de cétyle, les (méth)acrylates polyéthoxylés de cétéaryle, les (méth)acrylates polyéthoxylés de stéaryle, les (méth)acrylates polyéthoxylés d'arachidyle, les (méth)acrylates polyéthoxylés de béhényle, les (méth)acrylates polyéthoxylés de lauryle, les (méth)acrylates polyéthoxylés de cérotyle, les (méth)acrylates polyéthoxylés de montanyle, les (méth)acrylates polyéthoxylés de mélissyle, les (méth)acrylates polyéthoxylés de laccéryle, les (méth)acrylates polyéthoxylés de 2,4,6-tri(1'-phényléthyl)phényle, les (méth)acrylates polyéthoxylés de l'huile de ricin hydrogénée, les (méth)acrylates polyéthoxylés de canola, les (méth)acrylates polyéthoxylés du cholestérol et leurs mélanges, où la portion polyéthoxylée du monomère comprend de 5 à 100, de préférence de 10 à 80, et mieux de 15 à 60 unités oxydes d'éthylène.

9. Composition selon l'une quelconque des revendications précédentes, **caractérisée en ce que** le ou les monomères vinyliques associatifs représentent de 0,001 à 25 % en poids, de préférence de 0,01 à 15 % en poids du mélange de monomères.

10. Composition selon l'une quelconque des revendications précédentes, **caractérisée en ce que** le mélange de monomères comprend un ou plusieurs monomères tensioactifs vinyliques semi-hydrophobes d) choisis parmi les composés de formules (IV) ou (V) : 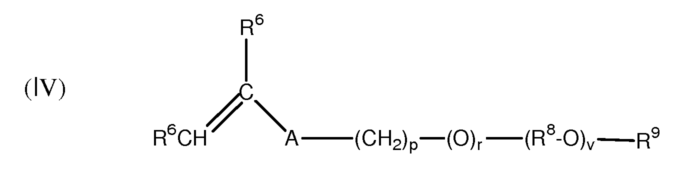
(V) D-A-(CH₂)ₚ-(O)ᵣ-(R⁸-O)ᵥ-R⁹
où, dans chaque formule (IV) ou (V),
chaque R⁶ représente indépendamment H, un alkyle en C₁-C₃₀, -C(O)OH, ou C(O)OR⁷ ;
R⁷ est un alkyle en C₁-C₃₀ ;
A est un groupe -CH₂C(O)O-, -C(O)O-, -O-, -CH₂O, -NHC(O)NH-, - C(O)NH-, -Ar-(CE₂)_{z}-NHC(O)O-, -Ar-(CE₂)_{z} -NHC(O)NH-, ou -CH₂CH₂NHC(O)- ;
Ar est un groupe aryle divalent ;
E est H ou un groupe méthyle ;
z est égal à 0 ou 1 ;
p est un entier variant de 0 à 30 ;
r est égal à 0 ou 1, à la condition que lorsque p est égal à 0, r est égal à 0, et lorsque p varie de 1 à 30, r est égal 1,
(R₈-O)ᵥ est un polyoxyalkylène qui est homopolymère, un copolymère aléatoire, ou un copolymère à blocs avec des unités oxyalkylène en C₂-C₄, où R⁸ est C₂H₄, C₃H₆, C₄H₈, ou leurs mélanges, et v est un entier variant de 5 à 250 ;
R⁹ est H ou un alkyle en C₁-C₄;
D est un alcényle en C₈-C₃₀ ou un alcényle en C₈-C₃₀ substitué par un groupe carboxy.

11. Composition selon la revendication 10 **caractérisée en ce que** le mélange de monomères comprend un ou plusieurs monomères tensioactifs vinyliques semi-hydrophobes ayant l'une des formules suivantes :
CH₂=CH-O(CH₂)ₐO(C₃H₆O)_{b}(C₂H₄O)_{c}H
ou
CH₂=CHCH₂O(C₃H₆O)_{d}(C₂H₄O)ₑH;
où
a est égal à 2, 3, ou 4 ;
b est un entier variant de 1 à 10 ;
c est un entier variant de 5 à 50 ;
d est un entier variant de 1 à 10 ; et
e est un entier variant de 5 à 50.

12. Composition selon l'une quelconque des revendications précédentes, **caractérisée en ce que** le ou les monomères tensioactifs vinyliques semi-hydrophobes représentent de 0 à 25 % en poids, de préférence de 0,1 à 10 % en poids du mélange de monomères.

13. Composition selon l'une quelconque des revendications précédentes, **caractérisée en ce que** le ou les monomères vinyliques non ioniques hydroxylés d) sont choisis parmi les (méth)acrylates d'hydroxyalkyle en C₁-C₆, les (hydroxyalkyl en C₁-C₄)(méth)acrylamides, et leurs mélanges.

14. Composition selon la revendication 13, **caractérisée en ce que** le monomère vinylique non ionique hydroxylé est le méthacrylate de 2-hydroxyéthyle.

15. Composition selon l'une quelconque des revendications précédentes, **caractérisée en ce que** le ou les monomères vinyliques non ioniques hydroxylés représentent de préférence de 0,01 à 10 % en poids du mélange de monomères.

16. Composition selon la revendication précédente **caractérisée en ce que** le mélange de monomères comprend, par rapport au poids total du mélange de monomères :
a) de 20 à 60 % en poids du ou des monomères vinyliques substitués par un ou plusieurs groupes amino,
b) de 20 à 70 % en poids du ou des monomères vinyliques non ioniques hydrophobes,
c) de 0,01 à 15 % en poids du ou des monomères vinyliques associatifs,
d) de 0,1 à 10 % en poids du ou des monomères tensioactifs vinyliques semi-hydrophobes,
e) de 0,01 à 10 % en poids du ou des monomères vinyliques non ioniques hydroxylés,
f) de 0,001 à 5 % en poids du ou des monomères réticulants, et
g) de 0,001 à 10% en poids du ou des agents de transfert de chaîne,
h) de 0 à 2 % en poids du ou des stabilisateurs polymériques.

17. Composition selon l'une quelconque des revendications précédentes **caractérisée en ce que** ledit mélange de monomères comprend :
- un méthacrylate de di(alkyl en C₁-C₄)amino(alkyle en C₁-C₆),
- un ou plusieurs esters d'alkyle en C₁-C₃₀ et de l'acide (méth)acrylique,
- un méthacrylate d'alkyle en C₁₀-C₃₀ polyéthoxylé avec 20 à 30 moles d'oxyde d'éthylène,
- un allyl éther de polyéthylèneglycol/polypropylèneglycol 30/5,
- un méthacrylate d'hydroxy(alkyle en C₂-C₆),
- un diméthacrylate d'éthylèneglycol.

18. Composition selon l'une quelconque des revendications précédentes, **caractérisée en ce que** le ou les polymères cationiques (i) sont présents à une concentration allant de 0,01 à 10% en poids par rapport au poids total de la composition, de préférence de 0,05 à 5% en poids et mieux de 0,1 à 1% en poids.

19. Composition selon l'une quelconque des revendications précédentes, **caractérisée par le fait que** les amidons sont choisis parmi les amidons de maïs, de riz, de manioc, d'orge, de pomme de terre, de blé, de sorgho, de pois.

20. Composition selon l'une quelconque des revendications précédentes, **caractérisée par le fait que** l'amidon est modifié par voie chimique et/ou physique.

21. Composition selon l'une quelconque des revendications précédentes, **caractérisée par le fait que** l'amidon est modifié par une ou plusieurs des réactions suivantes : prégélatinisation, oxydation, réticulation, estérification, étherification, amidification, traitements thermiques.

22. Composition selon l'une quelconque des revendications précédentes, **caractérisée par le fait que** l'amidon est modifié par au moins une estérification.

23. Composition selon l'une quelconque des revendications précédentes, **caractérisée par le fait que** l'amidon est choisi parmi les phosphates d'amidon.

24. Composition selon l'une quelconque des revendications précédentes, **caractérisée par le fait que** l'amidon est choisi parmi les amidons amphotères.

25. Composition selon l'une quelconque des revendications précédentes, **caractérisée par le fait que** le ou les amidons sont présents dans les composition de l'invention à une concentration allant de 0,1 % à 20 % en poids, de préférence de 0,5 % à 15% en poids par rapport au poids total de la composition finale.

26. Utilisation comme shampooing, lotion ou après-shampooing d'une composition telle que définie dans l'une quelconque des revendications précédentes.

27. Procédé de traitement cosmétique des matières kératiniques en particulier des cheveux, **caractérisé par le fait qu'**il consiste à appliquer sur lesdites matières une composition telle que définie selon l'une quelconque des revendications 1 à 25 puis à effectuer éventuellement un rinçage à l'eau, après un éventuel temps de pause.
